# EUROPEAN PATENT APPLICATION

(11) **EP 1 684 221 A1**
(43) Date of publication of application: **26.07.2006**
(21) Application number: 04793239.7
(22) Date of filing: 29.10.2004
(51) Int. Cl.: G06Q 10/00

(54) **MEDICAL INFORMATION COMPUTERIZED SYSTEM, PROGRAM AND MEDIUM**

(30) Priority: 06.11.2003 JP 2003377325
(71) Applicant: Matsunaga, Atsushi, Osaka-shi, Osaka 5300-001 (JP)
(72) Inventor: Matsunaga, Atsushi, Osaka-shi, Osaka 5300-001 (JP)
(74) Representative: Einsel, Martin
(86) International application number: PCT/JP2004/016138
(87) International publication number: WO 2005/045720

(57) **Abstract**

[Problem]

The problem is to facilitate viewing of temporal correlation between patient's chief complaint and doctor's interview results associated with the patient's chief complaint.

[Means of Solution]

The temporal correlation can be viewed by providing an electronic medical information system equipped with a control server comprising an input means for inputting, among the information written on the chart, the patient's chief complaint information into a chief complaint information file and for inputting the doctor's consultation information associated with the patient's chief complaint information into a consultation information file; an accumulation means for accumulating the chief complaint information and consultation information; a calculation means for scoring, with respect to each date of consultation, the latest chief complaint information and consultation information input by the input means, and the past chief complaint information and consultation information accumulated by the accumulation means, respectively; a generation means for automatically generating, based on the scores, a list by which the temporal variation of the chief complaint information and consultation information can be viewed.

## Description

### Technical Field

The present invention relates to an electronic medical information system, electronic medical information programs, and computer-readable recording media storing the electronic medical information programs, for introducing electronic medical information such as medical charts, using a computer

### Background Art

Recently, needs for electronic documents, or a paperless workplace are growing due to spread of computers. In medical institutions, there are high needs mainly for reducing conventional paper-based charts, for which a variety of systems have been proposed.

Generally, the advantage expected by promoting a paperless workplace may result from reduced storage space of paper documents and facilitated search by creating a database. With the statutory five-year retention period of medical charts, and the rapidly-ballooning volume of charts due to recent increase in longevity and diversity of diseases, the above-mentioned general advantage of paperless workplace is also expected for medical charts.

The advantages expected by electronic charts may include, other than the general ones stated above, those intrinsic for charts, such as raising the efficiency of medical service by relieving the doctors of troublesome handling of patient information, and sharing medical information to help enhancement of EBM (Evidence Based Medicine), whereby improving the critical path.

For example, in response to the above-mentioned requirements, online medical information sharing/medical institution cooperation systems have been introduced (see patent literature 1, for example), which manage medical information in a server system provided in a center to facilitate shared use of necessary information and cooperation between medical institutions, using the server system provided in the center where medical institution terminals installed in a plurality of institutions such as medical institutions, clinics, inspection centers, university hospitals, hospital-clinic cooperation hospitals are connected to a network for connecting computers, mobile terminals and the like, the medical institution terminal comprising a receipt computer, electronic charts, an image scanner, a display, a communication monitor, and the server comprising a storage means for storing data transmitted from the medical institution terminals such as medical institution-related data, patient-related data records such as charts, data records with regard to the course of medical practice or treatment contents, data showing cases of diseases, and the like; a control means for performing data processing and medical clerical work such as billing; a display means, an input means, and an output means

The main purpose of the doctor for referring to a patient's chart is to trace temporal variation of the patient's health from consultation in the past. Appropriate consultation and optimal treatment or prescription can be performed only after tracing such temporal variation.

Thus, it must be guaranteed that the temporal variation can be traced when introducing electronic charts. In this regard, although the above-mentioned system can store and display data records such as the course of consultation and treatment contents, no concrete solving means about how they are displayed is described.

In order to solve such problems, for example, there have been proposed a system (see patent literature 2, for example) which can display a list of electronic charts in time series using date labels, and a system (see patent literature 3, for example) which can display each specific item such as consultation information, consultation content, order/treatment, and the like on a single screen page.
Patent literature 1: Japanese Patent Application Laid-Open No. 2002-149833 (description of claim 1)
Patent literature 2: Japanese Patent Application Laid-Open No. 2000-293594 (claim 2, Figs. 1 and 5)
Patent literature 3: Japanese Patent Application Laid-Open No. 2001-5890 (claim 1)

### Disclosure of the Invention

### Problems to be solved by the Invention

By the way, although a chart is supposed to have all the consultation contents written thereon without any omission, most doctors, in reality, tend to write only the remarkable points as a memo due to time constraints. Usually, a doctor prepares a therapeutic plan at the time of re-consultation, by turning over the charts to recall the past data, grasping interview results associated with the patient's chief complaint, i.e. correlation with consultation, and analyzing the temporal variation of the patient's health.

However, actually, the analysis must be performed by correlating, simultaneously, not only the facts written on the chart but also any kind of relevant data, other than those on the chart, such as conversation with the patient in the past, diagrams drawn by the doctor for the patient, photographs of inspection results, prescribed drugs or the like. It often happens that the doctor must turn over the charts and other documents many times, tracing back in his/her memory of the past.

Thus, it may be no exaggeration to say that the significance of introducing electronic charts decreases by half if the troublesome work of turning over the charts many times and simultaneously correlating relevant the data can not be reduced.

However, the conventional systems do not propose any means for reducing the above-mentioned troublesome work.

In other words, a system using date labels to display a list of electronic charts in time series can only present the date labels to be actually viewed as a list on the display screen, whereas specific contents can not be viewed unless a date label is selected or specified. Thus, it is hard to say the temporal variation of the patient's health can be viewed substantially.

In addition, with the system which can display each specific item such as practice information, consultation content, order/treatment and the like on a single screen page, although each of the information can be viewed on a single screen page, there has been a problem in that narrowing down the information may become adversely difficult due to diversity of information which can be viewed, while the above-mentioned correlation which is most necessary for temporal tracing can not be displayed.

Furthermore, the prior art can not display relevant data such as conversation with the patient in the past, diagrams drawn by the doctor for the patient, photographs of inspection results, prescribed drugs or the like, in conjunction with the correlation.

It is therefore an object of the present invention to provide, in view of the above-mentioned problems, an electronic medical information system which facilitates viewing of the patient's chief complaint and the doctor's interview results associated with the patient's chief complaint, i.e. temporal correlation with consultation, and facilitates viewing of charts and relevant information with regard to the charts.

### Means for Solving the Problems

In order to solve the above-mentioned problems, an electronic medical information system according to the present invention uses a computer to manage electronic medical information such as charts, the electronic medical information system being equipped with a control server comprising: an input means for inputting, among information written on the chart, patient's chief complaint information into a chief complaint information file and for inputting doctor's consultation information associated with the patient's chief complaint information into a consultation information file; an accumulation means for accumulating the chief complaint information and consultation information; a calculation means for scoring, with respect to each date of consultation, the latest chief complaint information and consultation information input by the input means, and the past chief complaint information and consultation information accumulated by the accumulation means, respectively; a generation means for automatically generating, based on the scores, a list by which the temporal variation of the chief complaint information and consultation information can be viewed; and a display means for displaying the list.

According to this configuration, the temporal correlation between the chief complaint information and consultation information ranging from the past to the present can be viewed by inputting the chief complaint information and consultation information.

Furthermore, the control server may comprise a display means for displaying the list to be viewable.

According to this configuration, the list can be viewed directly from the control server.

The control server may be configured to set and display a table in which a plurality of symptoms and degrees of respective symptoms are selectably listed, the input means of the chief complaint information and consultation information automatically inputs one or more relevant symptoms and degrees of symptoms, in response to the user operation of selecting them from the table into the chief complaint information file and the consultation information file, and the calculation means is configured to automatically calculate by aggregating relevant scores corresponding to the degree of the selected symptom, each of the degrees of symptoms having a pre-defined score assigned thereto.

The control server may be configured such that it displays, in conjunction with the table, an image simulating a human body with check buttons provided on major regions thereof, and when any one of the symptoms is selected, the check button corresponding to the region which is developing the selected symptom is automatically checked.

According to the configuration, locations of development of a plurality of symptoms can be viewed simultaneously on the screen.

The list generation means may be configured to automatically generate a graph by which temporal variation of the scores calculated by the calculation means can be viewed.

In this case, the graph may consist of, for example, two polygonal lines representing variation of the aggregated scores for the chief complaint and consultation, respectively, with chief complaint and consultation date on the horizontal axis and calculated scores on the vertical axis

The list generation means may be configured to present the scores calculated by the calculation means in time series for respective symptoms of the chief complaint information and consultation information to allow comparison with the temporal variation of the graph.

According to the configuration, breakdown of the scores displayed in the graph can be viewed in conjunction with the graph.

The control server may comprise a display means for displaying an image simulating a human body in conjunction with the list and displaying, when symptoms of the chief complaint information and consultation information are input by the input means, link buttons at the region where the respective symptoms are developing on the image simulating the human body; a reading means for reading, from the chief complaint information or consultation information accumulated in the accumulation means, each information of the region displaying the link button when the displayed link button is pressed; and a display means for displaying the respective information which has been read out to be viewable.

According to this configuration, since the image simulating the human body which displays the location of development of symptoms is also displayed in the list, it becomes possible to recognize visually the location of development simultaneously on the screen, whereby facilitating viewing of details of chief complaint information and consultation information via link buttons.

The control server may comprise a generation means for generating a list so that, when a patient is receiving a consultation for a plurality of different diseases around the same period, the respective diseases can be viewed on the same screen in a distinguishable manner.

In this case, the diseases can be viewed on the same screen in a distinguishable manner by, for example, providing each of the polygonal lines with a different color according to the disease.

The control server may comprise: an input means for inputting electronic chart information composed of one or more of information as to disease name, chief complaint reported at the consultation, history of present illness, anamnesis, anamneses of family members, observations of the doctor, inspection, treatment, dosage, injection/instillation, next-reservation; a generation means for generating an electronic chart based on the input information; a storage means for storing the generated electronic chart into an electronic chart file; a reading means for setting and displaying, on the list, a link button associated with the electronic chart and reading the electronic chart from the electronic chart file when the link button is pressed; and a display means for displaying the electronic chart which has been read out to be viewable.

According to this configuration, both of the electronic chart and the list can be readily viewed together since they are linked to each other.

The input means for inputting the electronic chart information may be configured to input handwritten information either in an imaged or coded manner.

According to this configuration, the handwritten information for an electronic chart can be automatically imaged or coded.

The input means for inputting the electronic chart information may comprise an audio input means, such as a microphone, for inputting audio information, and an image input means such as a camera for inputting image information, wherein the electronic chart generation means may generate an electronic chart so that the audio information and image information can be replayed from the electronic chart as a part of the electronic chart.

According to this configuration, audio information and image information as well as text information can be recorded into the chart at the time of consultation.

If the patient did not appear on the day reserved in the next-reservation information which has been input by the input means of electronic chart information, the list generation means may generate the list after clearing the field for the day.

According to this arrangement, a gap occurs in the polygonal line in the graph, for example, since there is no score data for the no-show day in the dates on the horizontal axis.

The control server may comprise a reading means for reading, from the electronic chart file, one or more of information as to inspection, treatment, dosage, and injection/instillation; and an accumulation means for accumulating the information which has been read out; wherein the control server sets/displays a link button for each information on the list when said list generation means arranges the temporal variation of each of the accumulated information in time series so that it can be compared with the temporal variation of the said graph, reads corresponding information accumulated by said accumulation means upon pressing of any of the link buttons, and display the information which has been read out to be viewable.

According to this configuration, the temporal variation of the information as to inspection, treatment, dosage, injection/instillation, other than chief complaint information and consultation information, in the list can be simultaneously viewed on a single screen, and furthermore, details of each information can also be readily viewed, since the list is linked with each information.

The control server may comprise a storing means for storing, into a conventional chart file, a conventional chart converted into an electronic document format which precisely reproduces the original image; a reading means for reading, upon request for viewing a conventional chart file, the conventional chart requested for viewing from the conventional chart file; and a display means for displaying the conventional chart which has been read out to be viewable.

According to this configuration, if there exist, for a specific patient, a conventional chart and an electronic chart according to the present invention, both charts can be viewed with the control server.

Of the information recorded in the conventional chart file, the information corresponding to the electronic chart information may be accumulated by the accumulation means, and the list generation means may be configured to present the temporal variation of each information of the accumulated conventional chart in time series so that it can be compared with the temporal variation of the graph.

According to this configuration, the information recorded in a conventional chart can be also reflected in the list.

The input means of chief complaint information may be implemented by transmitting data to the control server from a communication terminal used by the doctor for consultation via LAN or WAN, or from the patient's communication terminal used by the patient via a communication network such as the Internet.

In addition, the input means of consultation information may be implemented by transmitting data from the consultation terminal to the control server.

According to these configurations, the patient can input his/her chief complaint information at home, and when a plurality of doctors is in attendance, such as in the case with a general hospital, consultation information by respective doctors can be input from respective consultation terminals used exclusively by respective doctors in attendance.

The patient's communication terminal may transmit, via a communication network such as the Internet to the control server, vital information including one or more of the patient's weight, blood pressure, frequency of meals, content of meals, taking exercise or not, sleeping hours, with the control server comprising an input means for receiving the transmitted vital information and inputting it into a vital information file, and a generation means for generating the list by arranging the vital information in time series so that it can be compared with the temporal variation of the graph.

According to this configuration, vital information, in addition to chief complaint information, consultation information and the like, can be easily complemented and referred to when preparing a therapeutic plan or the like.

The input means of electronic chart information may be implemented by transmitting data from the consultation terminal to the control server via LAN or WAN.

According to this arrangement, when a plurality of doctors are in attendance, such as in the case with a general hospital, respective doctors can input necessary information into the electronic chart from respective communication terminals used specifically by respective doctors for consultation.

The consultation terminal may comprise an input means for inputting the handwritten information in an imaged or coded form.

In addition, the consultation terminal may comprise the audio input means and the image input means.

According to these configurations, each of the doctors in attendance can respectively input handwritten information for an electronic chart, audio information, and image information when preparing an electronic chart from his/her consultation terminal.

Upon receiving a request, via a communication network such as a LAN, a WAN, or the Internet, for viewing the list or the respective information accumulated by the accumulation means from the consultation terminal, the patient's communication terminal, and a communication terminal of a viewer such as an inspection engineer, a dispensing pharmacy clerk, an ambulance crew, a paramedic, a doctor on duty, or a doctor of another hospital, the control server may be configured to display the list or each of the information to be viewable, within the authorized range, from the consultation terminal, the patient's communication terminal, and the viewer's communication terminal, respectively.

According to this configuration, related people other than the doctor in attendance or the patient can also view the list and each of the information from respective communication terminals within the authorized range.

The control server may be configured such that by letting an IC card reader/writer connected to a communication terminal read the IC card carried by a person permitted to use the communication terminal being one or more of the consultation terminal, the patient's communication terminal, and the viewer's communication terminal, the control server receives a signal requesting authorization via a communication network such as a LAN, a WAN, or the Internet and performs the authorization.

Furthermore, the control server may be configured such that, by letting the reader/writer connected to the communication terminal of a viewer such as an ambulance crew, a paramedic, or a doctor on duty read the IC card carried by the patient and the IC card carried by the ambulance crew, the paramedic, or the doctor on duty, in case of emergency, the control server receives and authorizes a signal requesting authorization via a communication network such as a LAN, a WAN, or the Internet, and displays at least information of the history of present illness and anamnesis so that they can be viewed with the viewer's communication terminal.

According to these configurations, an IC card can be used for the authorization. Particularly, even in the case where the patient's condition does not allow him or her to explain his/her medical condition, the ambulance crew, the paramedic, or the doctor on duty can obtain the patient's history information of present illness and anamnesis.

In order to solve the above-mentioned problems, the electronic medical information program according to the present invention is characterized in causing, for the purpose of introducing electronic medical information, a computer to function as: an input means for inputting a chief complaint information into the patient's chief complaint information file and for inputting a consultation information into the doctor's consultation information file; an accumulation means for accumulating the chief complaint information input into the chief complaint information file and the consultation information input into the consultation information file; a calculation means for calculating, for the purpose of scoring with respect to each date of consultation, the input latest chief complaint information and consultation information, and the accumulated past chief complaint information and consultation information, respectively; a generation means for automatically generating, based on the calculated scores, a list by which the temporal variation of the chief complaint information and consultation information can be viewed.

Furthermore, for the purpose of introducing electronic medical information, the electronic medical information program may cause a computer to function as a display means for displaying the list.

For the purpose of introducing electronic medical information, the electronic medical information program may cause a computer to function as: an input means for inputting the electronic chart information; a generation means for generating an electronic chart from the input electronic chart information; a storing means for storing the generated electronic chart in an electronic chart file; a reading means for setting/displaying link buttons associated with the electronic chart file on the list and reading, upon pressing of any of the link buttons, the electronic chart from the electronic chart file; and a display means for displaying the electronic chart which has been read out to be viewable.

For the purpose of introducing electronic medical information, the electronic medical information program may cause a computer to function as: a reading means for reading, from the electronic chart file, one or more of information as to inspection, treatment, dosage, and injection/instillation; an accumulation means for accumulating the information which has been read; a reading means for setting/displaying a link button for each information on the list when the list generation means arranged the temporal variation of each of the accumulated information in time series so that it can be compared with the temporal variation of the graph, and for reading the corresponding information accumulated by the accumulation means upon pressing of any of the link buttons; and a display means for displaying the information which has been read out to be viewable.

For the purpose of introducing electronic medical information, the electronic medical information program may cause a computer to function as: a storing means for storing, into a conventional chart file, the conventional chart converted into an electronic document format which precisely reproduces the original image; a reading means for reading, upon request for viewing the conventional chart file, a conventional chart requested for viewing from the conventional chart file; and a display means for displaying the conventional chart which has been read out to be viewable.

For the purpose of introducing electronic medical information, the electronic medical information program may cause a computer to function as: an accumulation means for accumulating the information corresponding to the electronic chart of the information recorded in the conventional chart file by the accumulation means; and a generation means for generating the list by arranging the temporal variation of each information of the accumulated conventional chart in time series so that it can be compared with the temporal variation of the graph.

For the purpose of introducing electronic medical information, the electronic medical information program may cause a computer to function as: an input means for receiving, upon transmission of vital information including at least one or more of the patient's weight, blood pressure, frequency of meals, content of meals, taking exercise or not, sleeping hours, from the patient's communication terminal to the computer via a communication network such as the Internet, the transmitted vital information and inputting it into a vital information file; and a generation means for generating the list by arranging the vital information in time series so that it can be compared with the temporal variation of the graph.

In order to solve the above-mentioned problems, the computer readable recording media according to the present invention is characterized in storing any of the electronic medical information programs.

### Effect of the Invention

As is apparent from the above descriptions, since the electronic medical information system according to the present invention enables viewing the temporal correlation between the patient's chief complaint and the doctor's interview results for patient's chief complaint, the doctor can view necessary information on a single screen without looking for charts, prescription, and inspection results in the past when consulting a patient, whereby appropriate and quick medical service can be promoted.

As a result, consultation time can be reduced as well as avoiding overlooking of the patient's chief complaint, whereby an effect of preventing medical errors can also be expected.

Furthermore, according to the system, since handwritten charts are accumulated, that is, conventional charts which existed before introducing the system can also be accumulated without discrimination, an integrated management including conventional charts can be realized.

Furthermore, since the charts generated by the system is difficult to tamper, and audio-recorded data of conversation with the patient and image data such as diagrams are recorded, they can be used as objective evidence in lawsuits relating to medical malpractices which are frequently occurring these days.

### Brief Description of the Drawings

- **Fig. 1**: illustrates a configuration of an electronic medical information system according to the present invention.
- **Fig. 2**: illustrates an exemplary initial screen display when accessing the system according to the present invention.
- **Fig. 3a**: illustrates an exemplary presentation of a first screen (interview sheet) when inputting chief complaint information.
- **Fig. 3b**: illustrates an exemplary presentation of a second screen (interview sheet) when inputting chief complaint information.
- **Fig. 3c**: illustrates an exemplary presentation of a third screen (interview sheet) when inputting chief complaint information.
- **Fig. 4a**: illustrates an exemplary presentation of a first screen (interview list) when inputting consultation information.
- **Fig. 4b**: illustrates an exemplary presentation of a second screen (interview list) when inputting consultation information.
- **Fig. 5a**: illustrates a first exemplary presentation of the electronic chart input screen when inputting electronic chart information.
- **Fig. 5b**: illustrates a second exemplary presentation of the electronic chart input screen when inputting electronic chart information.
- **Fig. 5c**: illustrates a third exemplary presentation of the electronic chart input screen when inputting electronic chart information.
- **Fig. 5d**: illustrates a fourth exemplary presentation of the electronic chart input screen when inputting electronic chart information.
- **Fig. 6a**: illustrates an exemplary presentation of a table screen listing names of diseases (customized).
- **Fig. 6b**: illustrates an exemplary presentation of a table screen listing names of diseases (in the order of Japanese alphabet).
- **Fig. 7**: illustrates an exemplary presentation of a handwriting input screen.
- **Fig. 8**: illustrates an exemplary presentation of an inspection search results screen.
- **Fig. 9**: illustrates an exemplary presentation of a table screen listing names of treatments.
- **Fig. 10a**: illustrates an exemplary presentation of a table screen listing names of medicines.

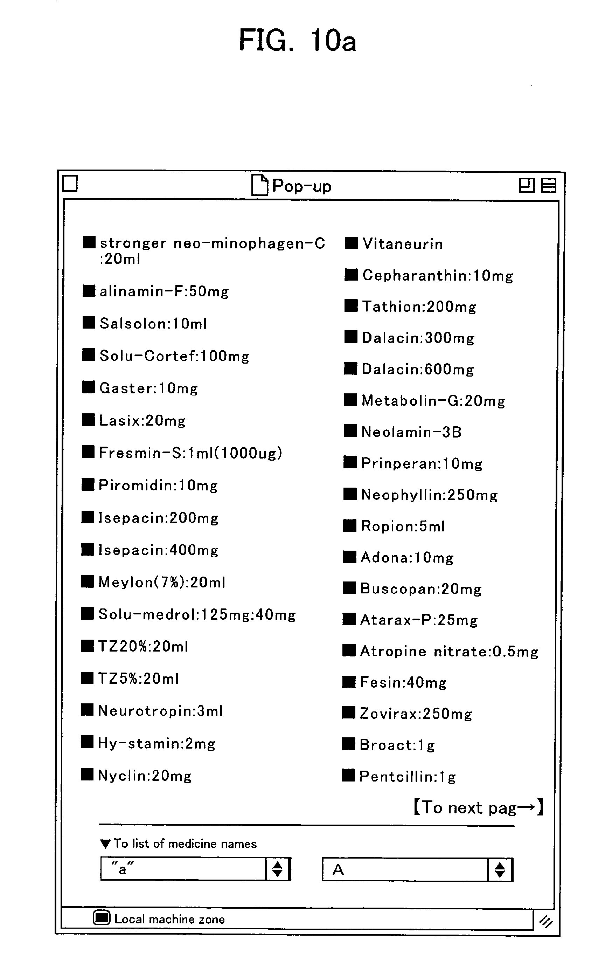

- **Fig. 10a**: illustrates an exemplary presentation of a table screen listing names of medicines.

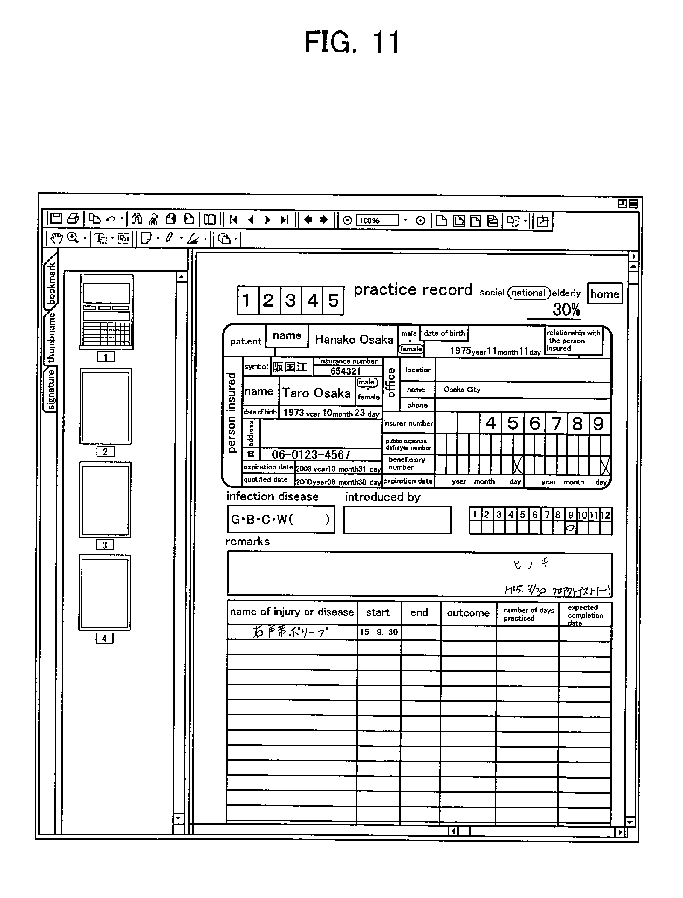

- **Fig. 11**: illustrates an exemplary presentation of a conventional chart screen.

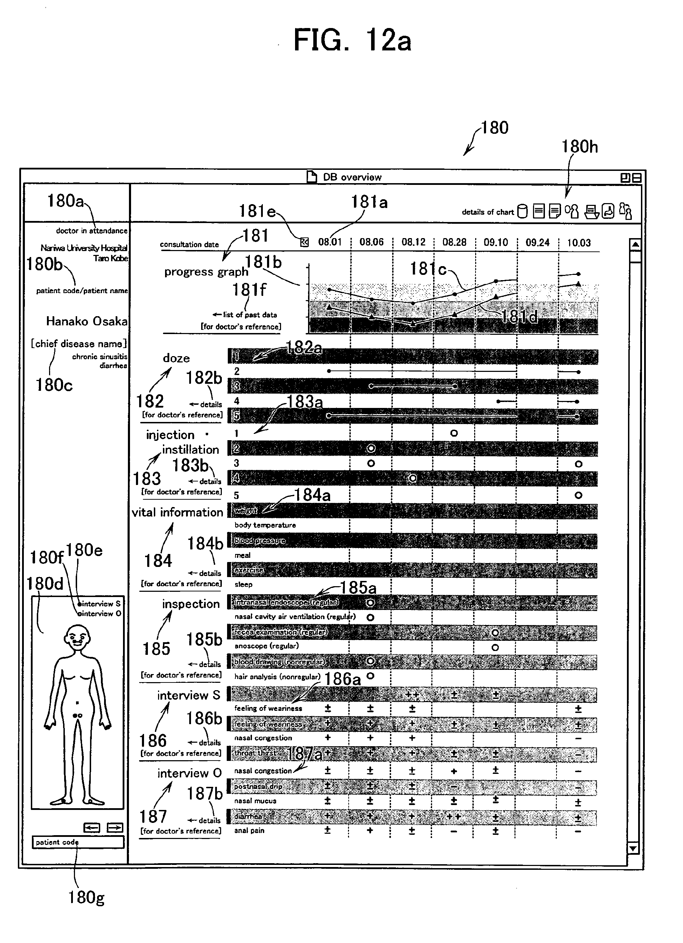

- **Fig. 12a**: illustrates an exemplary presentation of a database overview screen.

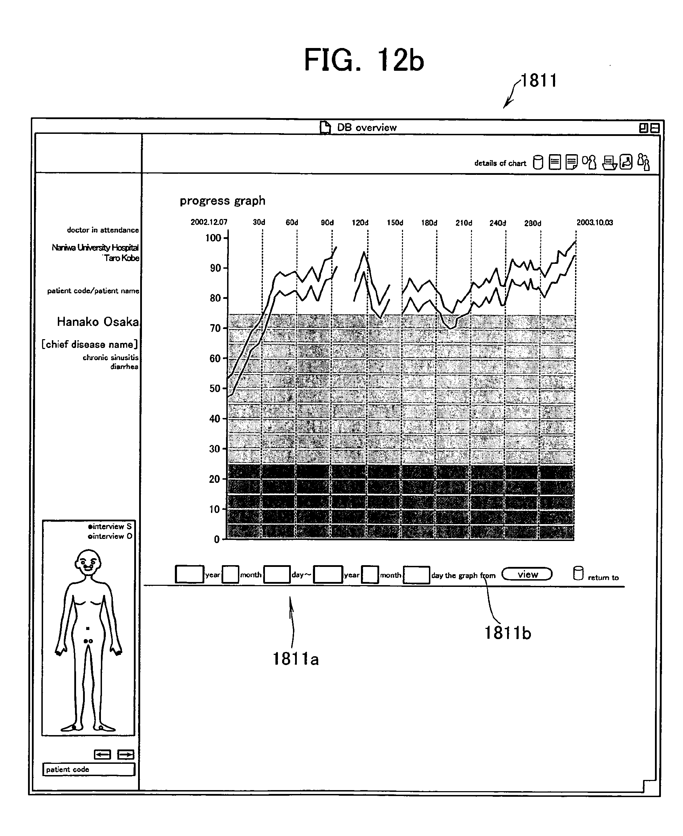

- **Fig. 12b**: illustrates an exemplary presentation of a list of progress graph screen.

### Explanation of Reference Numerals

- 1: control server
- 2: LAN/WAN
- 3: consultation terminal
- 4: Internet
- 5: patient's communication terminal
- 6: communication terminal of another hospital
- 7: communication terminal of an emergency hospital
- 8: communication terminal installed in an ambulance
- 11: electronic chart file
- 12: conventional chart file
- 13: chief complaint information file
- 14: vital information file
- 15: consultation information file
- 16: databases
- 17: database overview generation unit

### Best Modes for Carrying Out the Invention

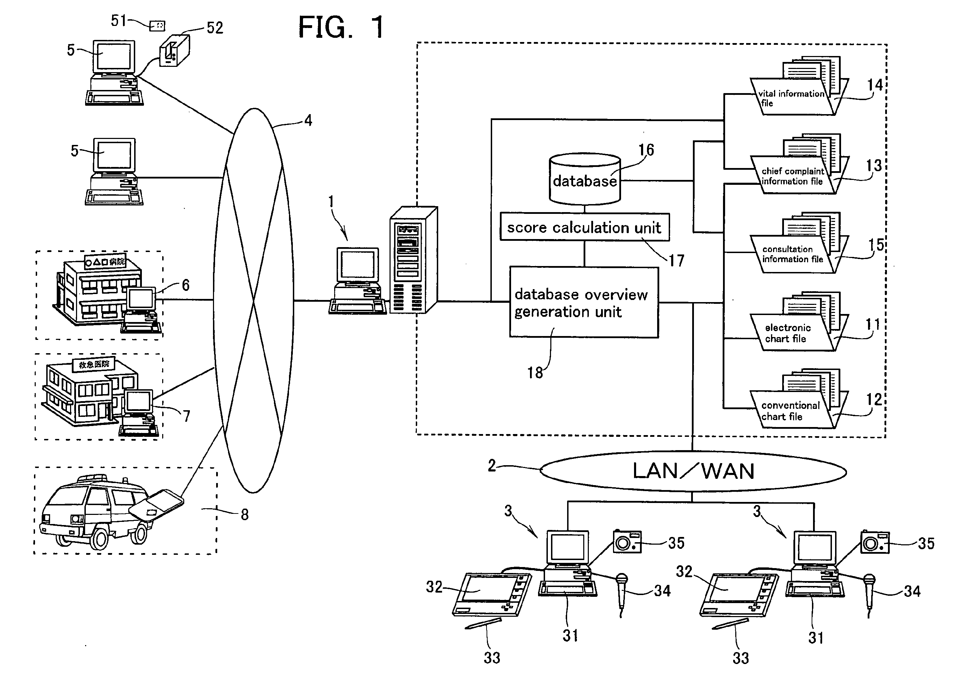

**Fig. 1** shows an example of the system configuration according to the present invention. Numeral 1 indicates a control server which mainly performs the tasks of transmitting and receiving various medical information to/from other communication terminals, rendering and storing the medical information into a file, creating a database of the medical information, and generating a list of data, according to the database.

The control server 1 may be directly used by a private-practice doctor, for example, as an input/output unit for each of the following medical information, however, when there is a plurality of doctors, it has one or more consultation terminals 3 connected thereto as client terminals for respective doctors in attendance via an in-hospital LAN/WAN 2.

The consultation terminal 3 in the present embodiment has a terminal unit 31 with a liquid crystal tablet 32 for handwriting input, an input pen 33, a microphone 34 for audio input, and a camera 35 for image taking connected thereto. The consultation terminal 3 is not limited to the present embodiment provided that it is capable of handling input and output of medical information, handwritten input, audio input, and image input.

An electronic chart of each patient generated by inputting relevant information from the consultation terminal 3 via the LAN/WAN 2 is stored into an electronic chart file 11 within the control server 1. The consultation terminal 3 comprises, as described above, a communication terminal 31 including a display unit, with a liquid crystal tablet 32, a microphone 34, and a camera 35 connected thereto.

The liquid crystal tablet 32 has an input pen 33 connected thereto. If a doctor inputs characters and the like in handwriting on the liquid crystal tablet 32 using the input pen 33 when generating an electronic chart, the input data in handwriting will be recorded on an electronic chart in situ. In the present embodiment, although a case has been described wherein input handwritten data is imaged and recorded on an electronic chart in situ, the input handwritten data may be coded to be recorded on the electronic chart.

In addition, if conversation between the doctor and the patient is recorded with the microphone 34, or a diagram drawn by the doctor for the patient for explanation purposes is photographed with a camera 35, the recorded audio data and the photographed image data will be recorded as a part of the electronic chart from the communication terminal 31 via the LAN/WAN 2.

Conventional charts such as those handwritten, which existed before the system according to the present invention is introduced, will be stored in a conventional chart file 12 within the control server 1, after converted into an electronic document format such as pdf, which can reproduce the original image precisely.

The control server 1 is also connected to external client terminals via the Internet 4, i.e., communication terminals for viewers such as a communication terminal 6 of another hospital, a communication terminal 7 of an emergency hospital, and a communication terminal 8 installed in the ambulance, as well as the patient's communication terminal 5.

Note that, the communication terminal 5 for the patient may have a reader/writer 52 of an IC card 51 connected thereto. In this case, the IC card 51 may be connected to the control server 1 by being read with the reader/writer 52.

The chief complaint information and vital information are transmitted to the control server 1 from the patient's communication terminal 5 via the Internet 4. Here, chief complaint information is information relating to symptoms reported by the patient, and vital information is information of the data recorded by the patient about predefined items (for example, weight, sleeping hours, content of meals).

The control server 1 stores the received chief complaint information and the vital information to a chief complaint information file 13 and a vital information file 14, respectively.

In addition, the chief complaint information may be input by a patient visiting the hospital from an in-hospital dedicated terminal (not shown), or input as data of the interview sheet handwritten by a nurse or a doctor according to direct interview to the patient, then stored into the chief complaint information file 13.

On the other hand, the consultation information is input from the consultation terminal 3 and stored into a consultation information file 15 of the control server 1 via the LAN/WAN 2. Here, consultation information means information relating to the result of the doctor's interview to the patient.

The control server 1 extracts data from the electronic chart file 11, the chief complaint information file 13, the vital information file 14, and the consultation information file 15, and accumulates each data in a database 16. Note that data of the conventional chart file 12 is directly input into the database 16 from the control server 1 or the consultation terminal 3 by an operator, for example.

The control server 1 generates a database overview using a database overview generation unit 18 based on each data accumulated in the database 16. At this timing, the score calculation unit 17 calculates, from the chief complaint information and consultation information, a score according to the degree of symptom and provides the calculation result to the database overview generation unit 18.

The control server 1 can be connected to the patient's communication terminal 5 via a communication network 4 such as the Internet. The patient's communication terminal 5 may have a reader/writer 52 of an IC card 51 connected thereto as described above. In this case, the IC card 51 may be connected to the control server 1 by being read with the reader/writer 52.

In addition, the control server 1 can also be connected to communication terminals for viewers such as a communication terminal 6 of another hospital which is different from the one to which the attending doctor belongs, a communication terminal 7 of an emergency hospital, or a communication terminal 8 installed in the ambulance, via the communication network 4.

Particularly, even in the case where the patient's condition does not allow him/her to explain his/her medical condition, necessary information for emergency care from the patient's history of illness can be obtained provided that the patient is carrying the IC card 51, by reading the IC card 51 and the IC card of the ambulance crew, the paramedic, or the doctor on duty of the emergency hospital with a reader/writer (not shown) installed in the emergency hospital 7 or the ambulance 8.

Here, a hand-held terminal may be used in the communication from the communication terminal 7 installed in the ambulance and, in this case, the ambulance 7 and the communication network 4 may be connected through a mobile communication network (not shown).

The control server 1, upon receiving, from the consultation terminal 3, a request of viewing the database overview generated by the database overview generation unit 15, displays it on the display unit of the consultation terminal 3. Details of each data accumulated in the database 16, electronic files stored in the electronic chart file 11, conventional charts stored in the conventional chart file 12, chief complaint information stored in the chief complaint information file, and vital information stored in the vital file 14 can also be accessed from the database overview displayed on the display unit of consultation terminal 3.

In addition, the database overview can be viewed from the patient's communication terminal 5, and communication terminals for viewers such as the communication terminal 6 of another hospital, the communication terminal 7 of an emergency hospital, and the communication terminal 8 installed in the ambulance, within an authorized range. Incidentally, the vital information and chief complaint information can also be viewed from the patient's communication terminal 5.

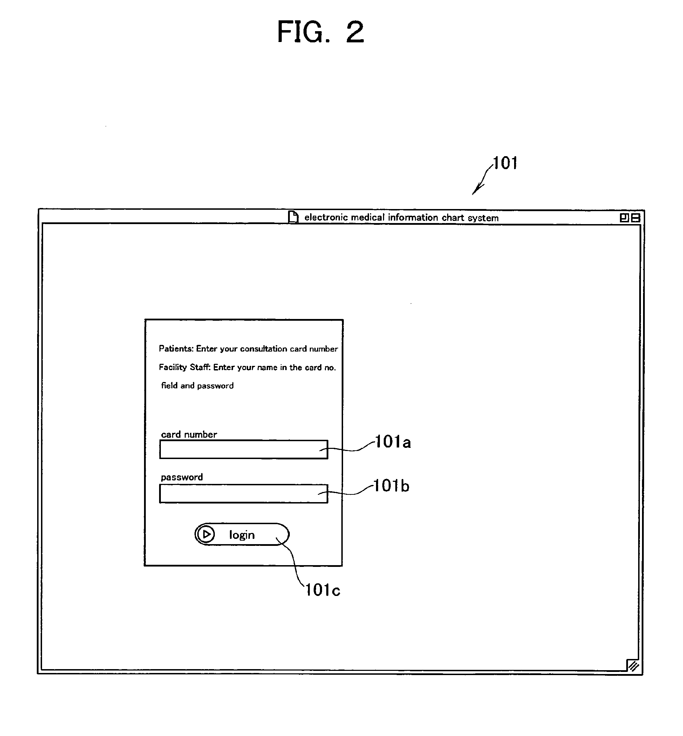

**Fig. 2** illustrates an exemplary presentation of an initial screen 101 when accessing the system according to the present invention from the control server 1, the patient's communication terminal 5, and communication terminals for viewers such as the communication terminal 6 of another hospital, the communication terminal 7 of an emergency hospital, and the communication terminal 8 installed in the ambulance.

In this example, a card number input field 101 a and a password input field 101 b are provided. Into the card number input field 101a, a re-visiting patient is supposed to input an assigned consultation card number and facility-belonging personnel (such as attending doctor, pharmacist, and administration staff) is supposed to input his/her name, from an input device (e.g. mouse or keyboard, the same hereinafter) of each communication terminal.

The initial screen 101 is not limited to the above arrangement, provided that security is guaranteed against access to charts or the like, which have personal information recorded thereon.

Incidentally, when accessing with the IC card 51, the screen jumps directly to the one which is desired to be displayed.

After the card number and password are input, and a login button 101c is specified (e.g. click on the mouse, the same hereinafter) for the input device of each communication terminal, a screen (not shown) is displayed for selecting an item desired to be accessed such as the database overview, the electronic chart, and vital information. Then, if a person checks an item that he or she wants to access, a screen for the desired item will be displayed.

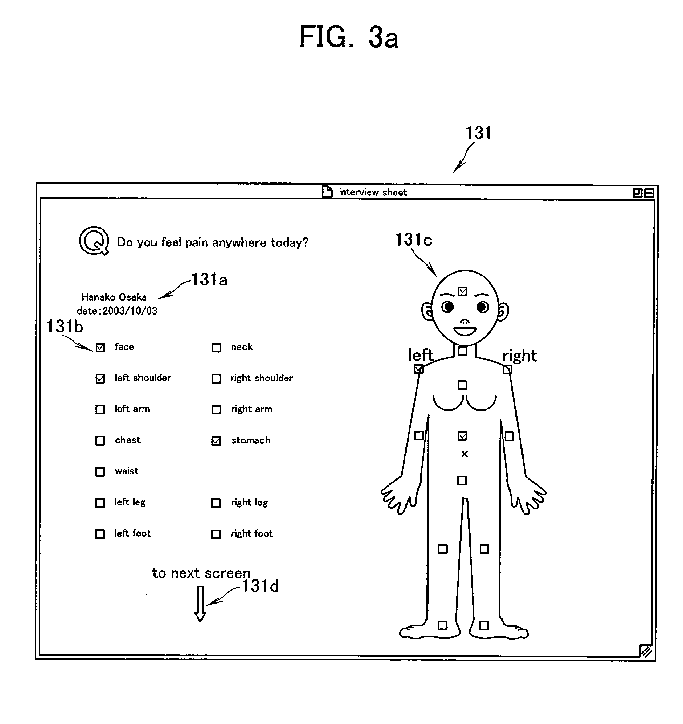

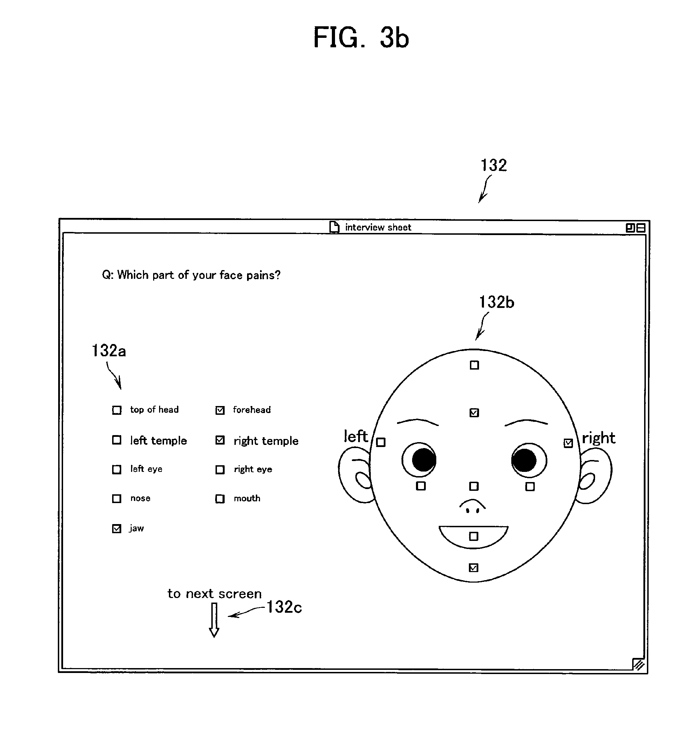

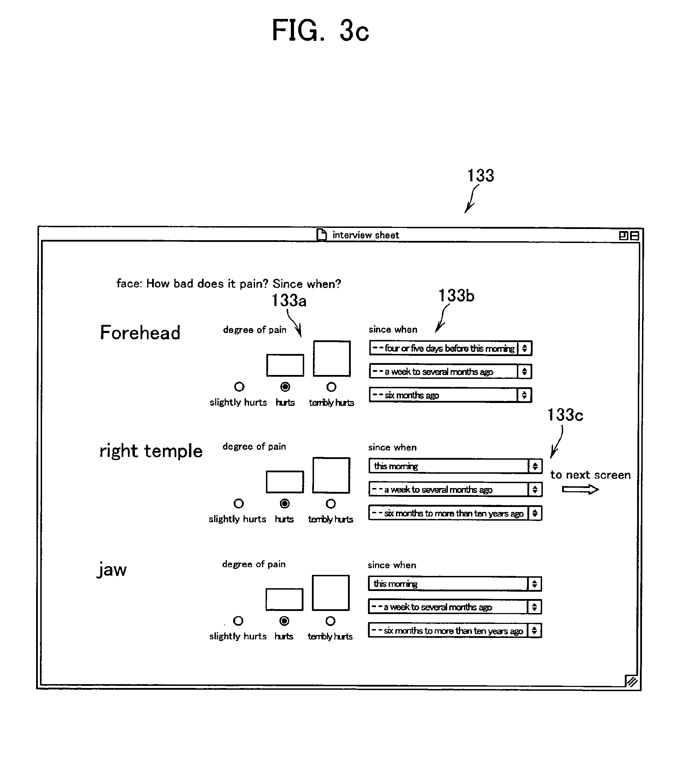

**Figs. 3a, 3b and 3c** are exemplary display screen (interview sheet) when inputting chief complaint information from the patient's communication terminal 5 or the consultation terminal 3. The chief complaint information input here is then stored into the chief complaint information file 13 in the control server 1.

A first screen 131 of the interview sheet of **Fig. 3a** displays the patient's name (Hanako Osaka) and the input date (2003/10/03) 131 a.

The first question "Q: Do you feel pain anywhere today?" is displayed, under which a table 131 b showing the names of body regions is displayed. The patient selects and specifies, from the input device of patient's communication terminal 5, one or more check buttons of the regions that give pain to the patient, among the regions displayed in the table 131 b. This case shows that check buttons of the face, the left shoulder and the stomach are selected.

Subsequent to the specification mentioned above, the check buttons of the corresponding regions on the simulated human body displayed on the right side of the screen, as shown in Fig. 131c, are also specified automatically.

When answers (specification of check buttons) to the first question are given, an arrow 131 d pointing to the second screen is specified to advance to the next screen.

**Fig. 3b** is an exemplary second screen 132 displayed by specifying the to-the-second-screen arrow 131d of Fig. 3a. In the second screen 132, sections with regard to each of the plurality of regions specified in the first screen 131 are questioned in more detail. In the present embodiment, a screen asking the detailed section on the face is shown. Thus, a question "Q: Which part of your face pains?" is displayed, under which a table 132a showing the names of the regions of the face is displayed. The patient selects and specifies, from the input device of patient's communication terminal 5, one or more check buttons of the regions that give pain to the patient, among the regions displayed in the table 132a. In the present embodiment, the forehead, the right temple, and the jaw are shown as specified.

Subsequent to the specification mentioned above, the check buttons of the corresponding regions on the simulated face displayed on the right side of the screen, as shown in Fig. 132b, are also specified automatically.

When answers (specification of check buttons) to the above question are given, an arrow 132c pointing to the third screen is specified to advance to the next screen.

**Fig. 3c** is an exemplary third screen 133 displayed by specifying the to-the-third-screen arrow 132c of Fig. 3b.

The third screen 133 asks questions about the degree of pain and duration of continuous pain for each of the regions of the face specified in the second screen 132. That is, a question "face: How bad does it pain? Since when?" is displayed, under which names of the regions of the face specified are displayed. In this case, "the forehead, the right temple, the jaw" is displayed in accordance with the items specified in the second screen. For each of the regions, a degree-of-pain input field 133a and a duration-of-pain input field 133b are displayed.

In this example, there are three alternatives "slightly hurts", "hurts" and "terribly hurts" for the degree-of-pain input field 133a, of which a corresponding check button is specified from an input device of the patient's communication terminal 5. In the present embodiment, a state is shown where "hurts" is selected for all the regions. The selected degree of pain will be scored as described below.

The duration-of-pain input field 133b is divided into three categories according to the length of duration, each of which takes the form of a pop-up menu. In the present embodiment, the first category is a period of "between this morning and 4 to 5 days ago", and the selected period is displayed (not shown) by specifying, among "since this morning", "since one day ago", "since two days ago", "since three days ago", "since four days ago" and "since five days ago", the corresponding one of the buttons on the right. Selection may be performed in a similar manner for the second category which is a period between 1 week and several months ago, and the third category which is a period between 6 months and 10 or more years ago, as with the first category.

Subsequent to the selection of respective regions in the duration-of-pain input field 133b, an arrow 133c pointing to the next region display screen is specified.

When the to-next-region-display-screen arrow 133c is specified, similar processing will be performed for the regions other than the face selected in Fig. 3a, namely the left shoulder or the stomach, as with Figs. 3b and 3c.

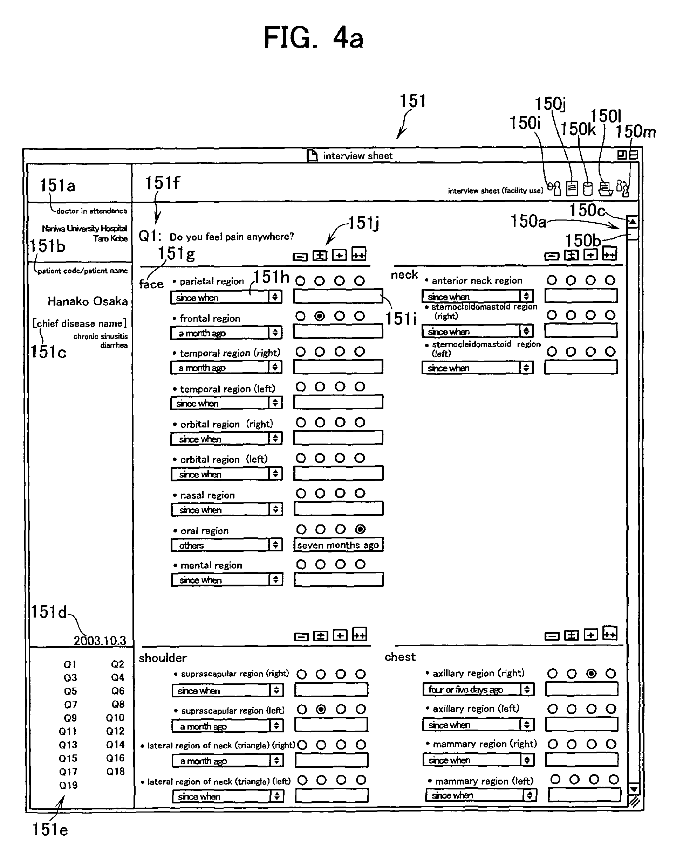

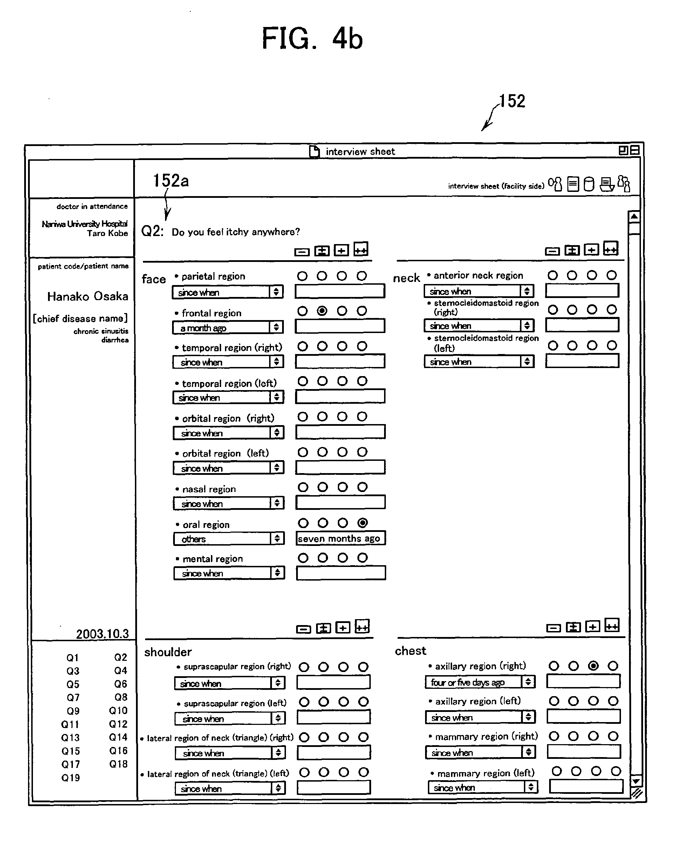

**Figs. 4a and 4b** are exemplary display screens (interview list) when inputting consultation information from the consultation terminal 3. The consultation information which is input here will be stored into the consultation information file 15 in the control server 1.

A scroll bar 150a is provided on the right side of the first screen 151 of the interview sheet of **Fig. 4a**, from which the viewer can move to the second screen **(Fig. 4b)** by dragging the scroll box 150b with the mouse or pushing the scroll arrow 150c down with the mouse.

On the left side of the first screen 151 of the interview sheet, there are provided a display field 151 a for the doctor in attendance ("Naniwa University Hospital, Taro Kobe" in this example), a display field 151b for the patient code/patient name ("Hanako Osaka" in this example), a chief disease name display field 151c ("chronic sinusitis, diarrhea" in this example), a display field 151d for the interview date ("2003.10.3" in this example), and a question selection button display field 151e (displayed from Q1 to Q19 in this example).

The interviews performed by doctors may generally have a certain pattern according to their medical specialty such as, internal medicine, surgery, otolaryngology or the like. Thus, the question selection button display field 151e may be provided with preliminarily patterned questions as a standard feature, for example, and customized according to the request of the doctor if he/she wants a question to be added to or deleted from the standard feature.

The doctor only has to select necessary questions according to the patient's chief complaint or the like from the question selection button display field 151e. The selection may be performed among the alternatives (in this case, Q1 to Q19) on the question selection button display field 151e from the input device of the consultation terminal 3.

In the present embodiment an exemplary display screen is shown wherein "Q1" and "Q2" are selected in Figs. 4a and 4b, respectively.

The question 151f of "Q1" of Fig. 4a is "Do you feel pain anywhere?" The pain-region display 151g displays, in the present embodiment, "the face" "the neck" "the shoulder" and "the chest". Each item in the pain-region display 151g has a detail display field 151 h provided thereto. The detail display field 151 h is a field for displaying the location, in further detail, for each of the regions displayed on the region display 151g, wherein, for example, there are nine items to be displayed in detail, from "the parietal region" to "the mental region" in the "face". Under each displayed item of the detail display field 151 h, a field for selecting the duration of pain is provided in the form of a pop-up menu. Thus, by specifying a button on the right side of this field, an alternative of periods is displayed (not shown) and a period is displayed in this field by selecting a corresponding period. For example, in the present embodiment, "since one month ago" is displayed as selected for "the frontal region of head" of the detail display field 151 h.

Next to the period selection field, there is provided a field 151i for inputting a specific period. If there is no corresponding period in the alternatives of the pop-up menu of the period selection field, a specific period may be directly input from the input device into the field 151i for inputting a specific period. For example, in the present embodiment, a period of "since seven months ago" is input into the field 151 for inputting the period for which pain is lasting for the "oral region" of the detail display field 151 h.

Corresponding to each of the displayed items of the detail display field 151 h, a consultation evaluation field 151j is provided adjacently. Each of the displayed items of the consultation display field can be evaluated into four ranks:"-", "±", "+" and "++". In other words, the corresponding medical condition among the four ranks for each of the displayed items may be selected and specified with the consultation terminal 3. For example, in the present embodiment, a state is shown wherein "±" is specified for "the frontal region". Each of the selected evaluation will be scored as described below.

Note that an icon which enables transferring to other pages is provided on the top right of the screen. In the present embodiment, an icon 150i representing interview sheets, an icon 150j representing electronic charts, an icon 150k representing the database overview, an icon 1501 representing conventional charts, and an icon 150m representing the top page are provided, but are not limited to these icons.

The second screen 152 of the interview sheet of Fig. 4b displays a question 152a of "Q2", i.e., "Do you feel itchy anywhere?" Since the arrangement of the screen is similar to that of Fig. 4a, description of each item is omitted.

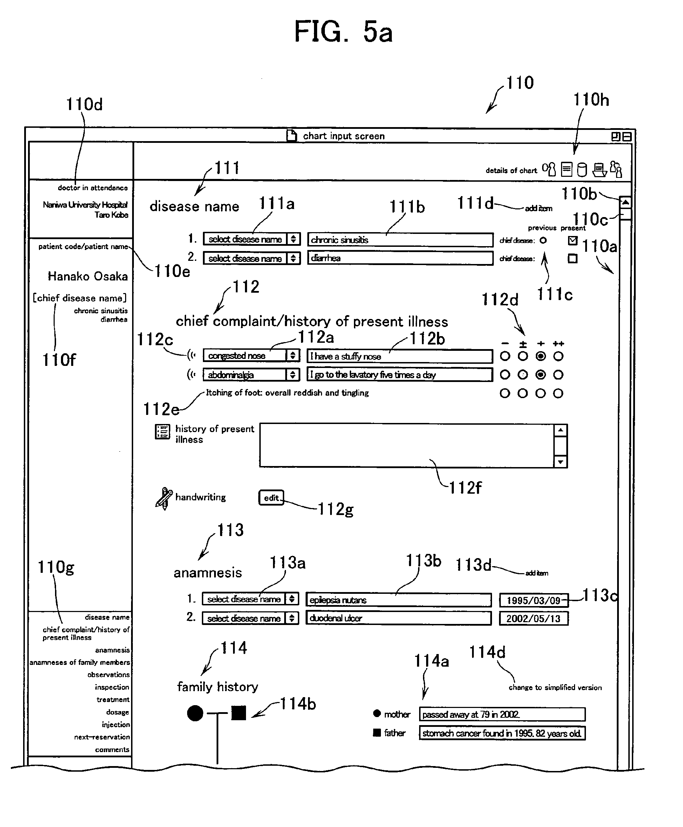

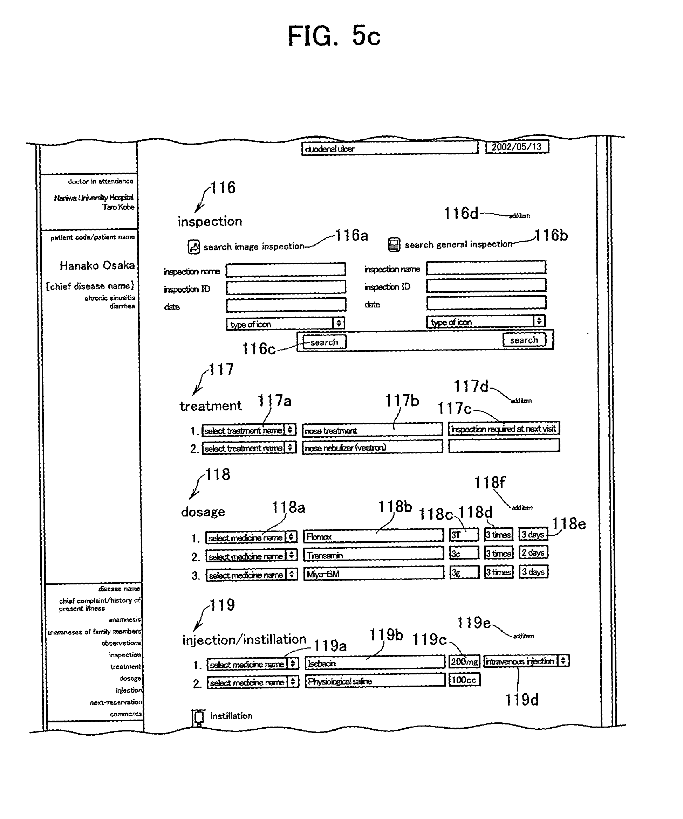

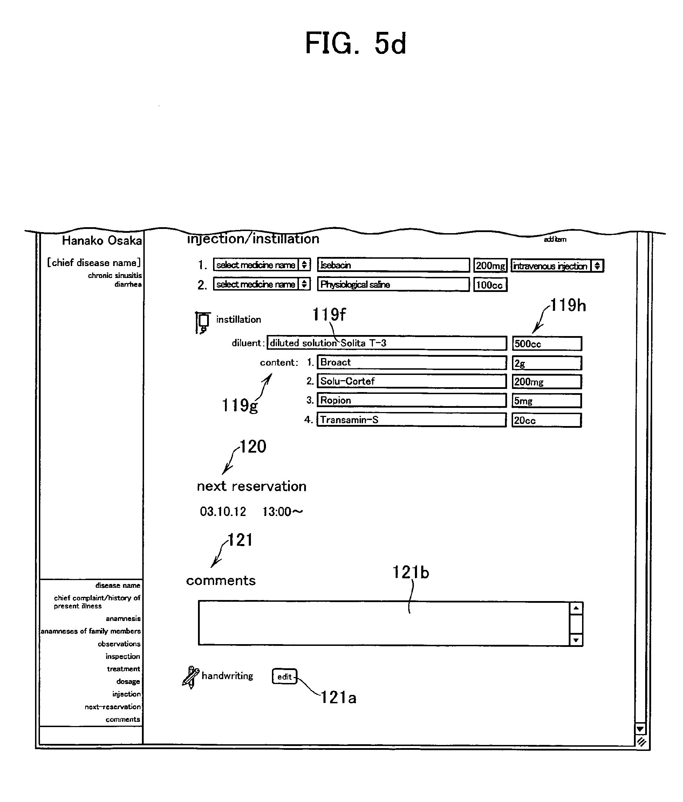

**Figs. 5a to 5d** are exemplary electronic chart input screen when inputting electronic chart information from the consultation terminal 3.

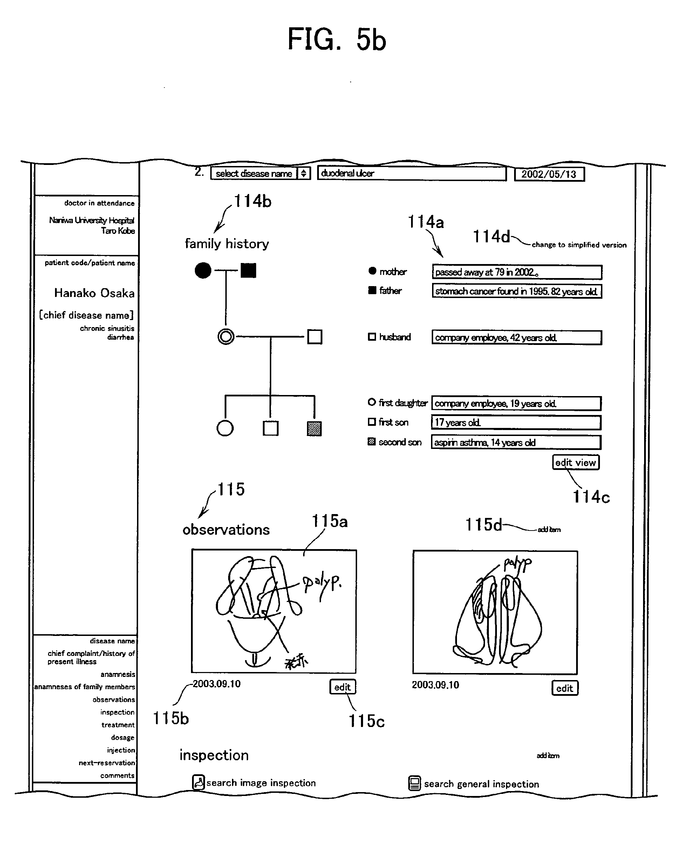

A scroll bar 110a is provided on the right side of the first input screen 110 of electronic chart of Fig. 5a, from which the viewer can move around the second to the fourth screens of Figs. 5b to 5d by dragging the scroll box 110b with the mouse, or pushing the scroll arrow 110c down with the mouse.

On the left side of the first input screen 110 of electronic chart, there are provided a doctor in attendance display field 110d ("Naniwa University Hospital, Taro Kobe" in this example), a patient code/patient name display field 110e ("Hanako Osaka" in this example), a chief disease name display field 110f ("chronic sinusitis, diarrhea" in this example), an electronic chart item display field 110g ("disease name", "chief complaint/history of present illness", "anamnesis", "anamnesis of family members", "findings", "inspection", "treatment", "dosage", "injection", "next-reservation" and "comments" in this example).

Incidentally, the viewer can move to the location of each item by clicking each item on the electronic chart item display field 110g with the mouse.

An icon menu 110h is displayed on the top right of the screen. Since the content of the menu is similar with the icon menu of Fig. 4a, its description is omitted.

By selecting a disease name from the disease name selection field 111a provided in the form of a pop-up menu in the disease name field group 111, the selected disease name is displayed in the disease name display field 111 b. In the present embodiment, an example is shown wherein "chronic sinusitis" and "diarrhea" are displayed in the disease name display field 111 b.

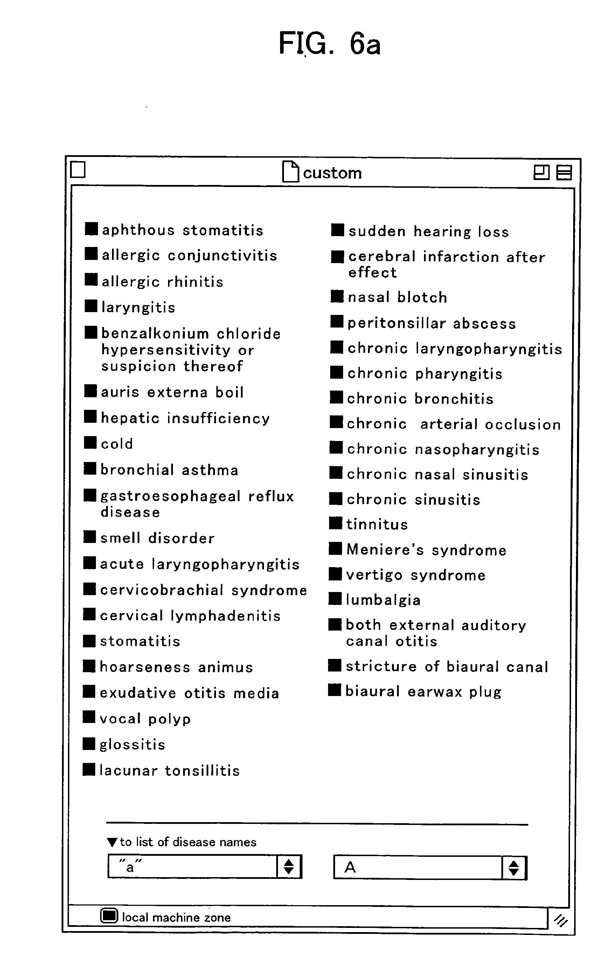

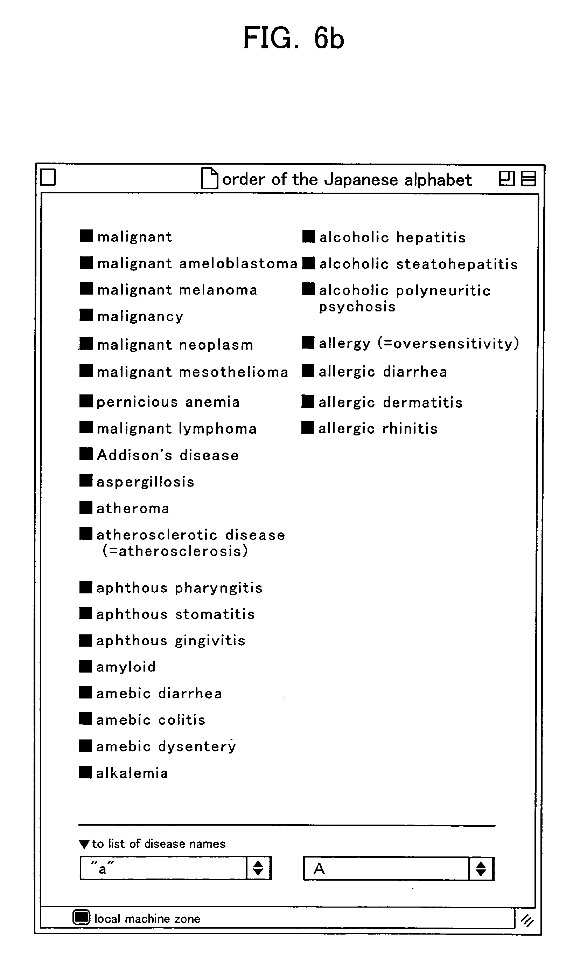

Here, the pop-up menu of the disease name selection field 111a can be customized by respective hospitals or doctors using this system, by selecting from the table listing the disease names shown in **Figs. 6a and 6b**. As shown in Fig. 6b, a pop-up menu for selecting a Japanese alphabet and a pop-up menu for selecting an alphabet are provided under the "list of disease names" at the bottom of the screen. In the present embodiment, an exemplary screen is shown where the Japanese alphabet 'a' is selected.

Returning to Fig. 5a, a chief disease display box 111c for indicating the chief disease when a plurality of disease names is displayed is provided next to the disease name display field 111b. In the present embodiment, the box is arranged so as to display the previous and the current chief disease, showing that "chronic sinusitis" is the chief disease for both occasions.

Incidentally, in the present embodiment, although two disease names are displayed, more disease names may be displayed by clicking on the additional item button 111 d with the mouse.

A chief complaint selection field 112a is provided in the chief complaint/history of present illness field group 112 so that the chief complaint can be selected in the form of a pop-up menu. This uses a chief complaint table (not shown). In the present embodiment, "congested nose" and "abdominalgia" are displayed in the chief complaint selection field 112a.

Next to the chief complaint selection field 112a, there is provided an audio information display field 112b in which conversation between the doctor in attendance and the patient is recorded. The audio information displayed in this audio information display field 112b is recorded with a microphone 34. The audio recorded with the microphone 34 is converted into text information and displayed by the audio information display field 112b. The audio information can be played back with the mouse click on the audio playback button 112c.

Here, the chief complaint selection field 112a and the audio playback information display field 112b correspond to the disease name displayed in the disease name display field 111 b. That is, in the present embodiment, "congested nose" of the chief complaint selection field 112a and "I have a stuffy nose" of the audio information display field 112b correspond to "chronic sinusitis" of the disease name display field 111b. In addition, "abdominalgia" of the chief complaint selection field 112a and "I go to the lavatory five times a day" of the audio information display field 112b correspond to "diarrhea" of the disease name display field 111 b.

A symptom evaluation field 112d for scoring the degree of symptom is provided next to the audio information display field 112b. The doctor in attendance is supposed to ask about the patient's complaint directly and evaluates the interview result into four ranks "-", "±", "+" and "++". The doctor in attendance only has to click the position of the degree of evaluation with a mouse or the like. In the present embodiment, an evaluation of "+" is provided for both of the two chief complaints "I go to the lavatory five times a day" and "I have a stuffy nose". In addition to the chief complaint information which has been preliminarily input using the patient's communication terminal 5 or the like, the patient's chief complaint can be grasped more precisely by the doctor in attendance having a conversation with the patient, listening to the patient's chief complaint, and recording it on the electronic chart.

In addition, if there is a patient's chief complaint other than the chief complaint corresponding to the disease name displayed in the disease name display field 111b, the additional chief complaint content can be displayed by inputting the chief complaint in the additional chief complaint display field 112e. In the present embodiment a chief complaint "Itching of foot: overall reddish and tingling" is added.

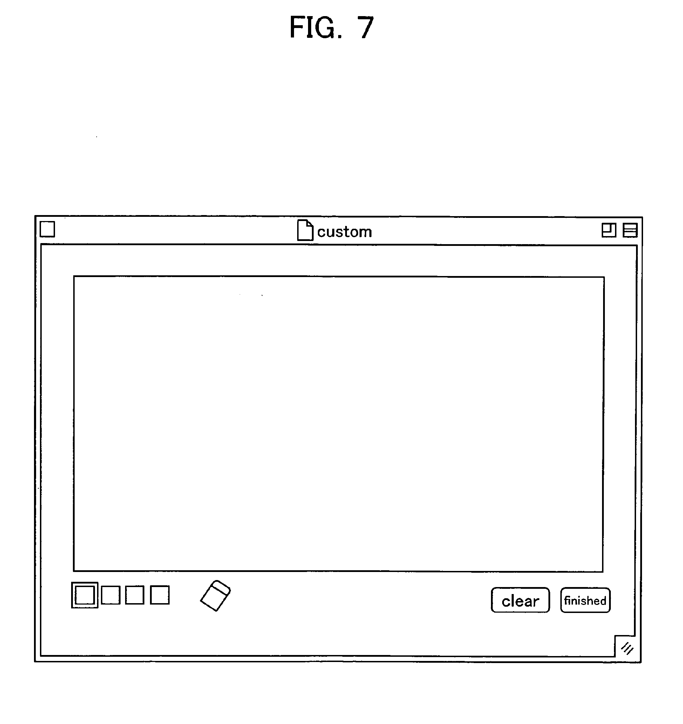

The doctor in attendance can describe particular observation, if any, about the history of present illness in the history of present illness field 112f. The description into the history of present illness field can be edited by clicking on the handwriting edition button 112g with the mouse to display a handwriting input screen shown in a° and handwriting the description on a liquid crystal tablet 32 with an input pen 33. After the input of handwritten description is finished, a completion button at the bottom right of Fig. 7 is clicked with the mouse, and then the input handwritten description is displayed in the history of present illness field 112f. To clear the handwritten description, a clear button at the bottom right of Fig. 7 may be clicked with the mouse.

The history of present illness field 112f may display handwritings either as images without change or as coded type fonts after conversion by means of a handwriting input device. If displayed as is handwritten, it is effective for preventing later tampering.

Returning to Fig. 5a, the anamnesis display field group 113 displays the patient's anamnesis that existed before ongoing consultation. The disease name selection field 113a in the anamnesis display field group 113 takes the form of a pop-up menu similar to that of the disease name selection field 111a, to select a disease name from the table listing the disease names shown in Figs. 6a and 6b. The selected disease name is displayed in the disease name display field 113b. In the present embodiment, "epilepsia nutans" and "duodenal ulcer" are displayed. To the immediate right of the disease name display field 113b, there is provided a date field 113c for inputting the dates on which respective anamneses had cured completely. In the present embodiment, the dates are "1995/03/09" for the "epilepsia nutans" and "2002/05/13" for the "duodenal ulcer".

Incidentally, the item addition button 113d may be clicked on with the mouse to add an item in the anamnesis display field 113.

The chief complaint/history of present illness field group 112 and the anamnesis display field group 113 may be arranged to be accessible from, for example, the communication terminal 7 of an emergency hospital, and the communication terminal 8 installed in an ambulance. When an emergency patient's condition does not allow him or her to explain his/her medical condition, the ambulance crew, the paramedic, or the doctor on duty of the emergency hospital can take appropriate measures by viewing the information described in the chief complaint/history of present illness field group 112 and the anamnesis display field group 113.

The family history field group 114 displays the anamneses of family members. The family history display field 114a describes the relationship of family members to the patient, and their respective anamneses, age, and the like of each of them. In addition, a mark indicating each family member is displayed on the left of each relationship. In the present embodiment, where the patient's mother is taken as an example, a mark is indicated in a black circle with a description "passed away at 79 in 2002".

A family tree 114b is displayed using the mark indicated for each family member in the family history display field 114a. To change the family tree 114b, an edition button 114c may be clicked on with the mouse to display an editing screen (not shown).

Here, a simplified display button 114d may be clicked on with the mouse to display only the family tree 114b.

The observations field group 115 includes an observations display field 115a which displays the observations of the doctor in attendance. In the present embodiment, a handwritten drawing by the doctor in attendance is photographed with a camera 35, and then taken into an electronic chart. An observation creation date field is provided at the bottom left of the observations display field 115a. In the present embodiment, the creation date of the observation is "2003.09.10". To edit the displayed content of the observations display field 115a, an edition button 115c may be clicked on with the mouse to display an editing screen (not shown). In addition, an item addition button 115d may be clicked on with the mouse when making addition to the observations display field 115a.

An inspection field group 116 is arranged so that the history of inspection which has been done so far can be searched. In the present embodiment, items to be searched are divided to an image inspection search field 116a and a general inspection search field 116b.

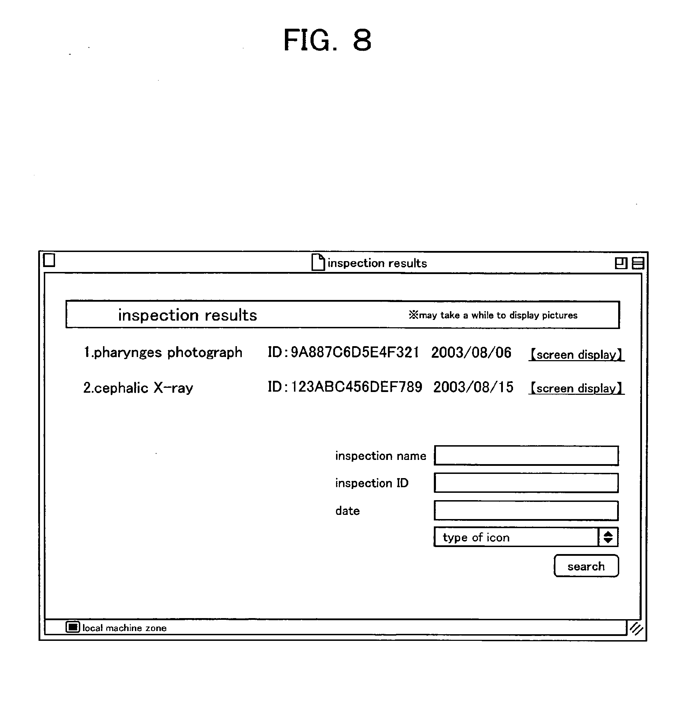

Respective search items are arranged to be searched by specifying "inspection name", "inspection ID", and "date". An exemplary search result screen display when the above "inspection name", "inspection ID", and "date" are specified and a search button 116c is clicked on with the mouse is shown in Fig. 8. Fig. 8 shows a search result of "pharynx photograph" for "inspection name", "9A887C6D5E4F321" for "inspection ID" and "2003/08/06" for "date", and a search result of "head X-ray" for "inspection name", "123ABC456DEF789" for "inspection ID" and "2003/08/15" for "date".
By clicking on the screen display button provided on the right of respective search results with the mouse, photograph of the pharynx, X-ray of the head and the like are displayed on screen (not shown).

Incidentally, at the bottom right of the screen, there are provided a field and a search button for specifying "inspection name", "inspection ID" and "date" so that still another type of inspection can be performed from the search results screen of **Fig. 8**.

If addition of a search item is desired, an item addition button 116d may be clicked on with the mouse.

Since image data such as X-ray or CT scanning can be efficiently referred to simultaneously with other items described in the electronic chart by recording the inspection field group 116 as a part of the electronic chart, judging the inspection results showing internal organ specificity, discovering or tracing abnormal shadows can be performed smoothly.

In a treatment field group 117, selecting a treatment name from a treatment name selection field 117a in the form of a pop-up menu displays the selected treatment name in a treatment name display field 117b. In the present embodiment, an example is shown wherein "nose treatment" and "nose nebulizer (vestron)" are displayed in the treatment name display field 117b.

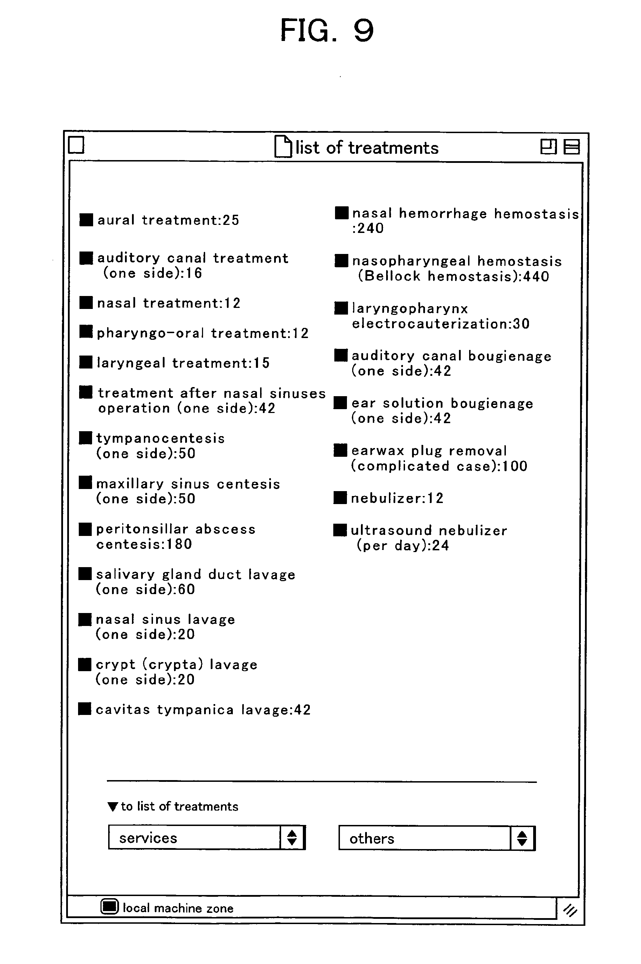

Here, the pop-up menu of the treatment name selection field 117a is created by selecting from a table listing treatment names as shown in **Fig. 9**. In the present embodiment, an exemplary table of treatment names for the otorhinolaryngology department is shown. To display a table of treatment names for other departments, the pop-up menu of the treatment list at the bottom of the screen may be clicked on and selected. The scores presented adjacent to respective treatment names of Fig. 9 are insurance scores. Thus, for example, the control server 1 may be configured to automatically calculate the insurance score using these insurance scores.

Immediately right to the treatment name display field 117b, there is provided a remark field 117c in which individual remarks relating to the treatment can be described. In the present embodiment, an example is shown wherein "inspection required at next visit" is described for "nose treatment" of the treatment name display field 117b.

If addition of a treatment item is desired, an item addition button 117d may be clicked on with the mouse.

In a dosage field group 118, by selecting a medicine name from a medicine name selection field 118a in the form of a pop-up menu, a name of the dosed medicine is displayed in a medicine name display field 118b. In the present embodiment, an example is shown wherein "Flomox" "Transamin" "Miya-BM" are displayed in the medicine name display field 118b.

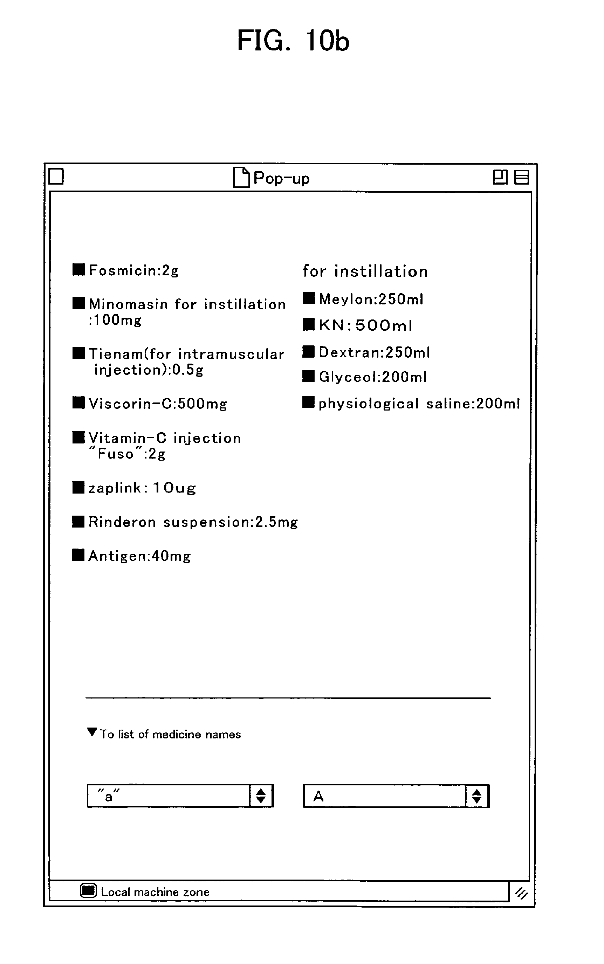

Incidentally, the pop-up menu of the medicine name selection field 118a is created by selecting from a table listing medicine names as shown in **Figs. 10a and 10b.** In the present embodiment, an exemplary table of treatment names for the otorhinolaryngology department is shown. To display a table of treatment names for other departments, the pop-up menu of the treatment list at the bottom of the screen may be clicked on and selected.

Immediately right to the medicine name display field 118b, there are provided a prescription amount display field 118c of the medicine, a dosage per day display field 118d, and a number of prescription days display field 118e. In the present embodiment, with the medicine name "flomox" taken as an example, "3T" in the prescription amount display field 118c, "3 times" in the dosage per day display field 118d, and "3 days" in the umber of prescription days display field 118e are shown, respectively.

Here, if addition of a treatment item is desired, an item addition button 118f may be clicked on with the mouse.

In an injection/instillation field group 119, by selecting a treatment name from an injection/instillation name selection field 119a in the form of a pop-up menu, the selected medicine name is displayed in an injection/instillation name display field 119b. In the present embodiment, an example is shown wherein "Isebacin" and "physiological saline" are shown in the injection/instillation name display field 119b.

Here, the pop-up menu of the injection/instillation name selection field 119a is created by selecting from a table listing medicine names as shown in Figs. 10a and 10b. In the present embodiment, an exemplary table of treatment names for the otorhinolaryngology department is shown. To display a table of treatment names for other departments, the pop-up menu of the treatment list at the bottom of the screen may be clicked on and selected.

Immediately right to the injection/instillation name display field 119b, there are provided a prescription amount display field 119c of the medicine and an injection method display field 119d. In the present embodiment, with the medicine name "Isebacin" taken as an example, "200 mg" in the prescription amount display field 119c and "intravenous injection" in the injection method display field 118d are shown, respectively.

Here, if addition of a treatment item is desired, an item addition button 119e may be clicked on with the mouse.

With regard to instillation, a dilution solution display field 119f, a constituent (content) display field 119g, and an amount of dilution solution and amount of content display field 119h are provided. In the present embodiment, "Solita T-3" in the dilution solution display field 119f, "Broact", "Solu-Cortef", "Ropion" and "Transamin-S" in the constituent (content) display field 119g are displayed respectively, while "500cc" for dilution solution and "2g" for Broact are displayed in the amount of dilution solution and amount of content display field 119h.

A next reservation display field 120 displays a date and time of the patient's next reservation. In the present embodiment an example is shown wherein "03.10.12 13:00-" is displayed for the date and time of the next reservation.

Upon display of the date and time of the next reservation, the date is automatically described on the list (for example, along the horizontal axis in the case of the above-mentioned graph).

A comment field group 121 includes a handwriting edition button 121a and a comment display field 121 b. By clicking on the handwriting edition button 121 a with the mouse, a handwriting input screen as shown in Fig. 7 is displayed, allowing edition by handwriting into the liquid crystal tablet 32 using the input pen 33. After the handwriting of items to be input is completed, a completion button at the bottom right of Fig. 7 is clicked on with the mouse to display the handwriting input items on the comment display field 121b. To clear the description of handwritten input items, the clear button at the bottom right of Fig. 7 may be clicked on with the mouse.

The handwriting input device may display handwritings either as images without change or as coded type fonts, similar to the case of history of present illness field 112f.

**Fig. 11** illustrates an exemplary screen displaying a conventional chart which is converted into an electronic document format precisely reproducing the original image, and stored in the conventional chart file 12 of the control server 1. Here, conversion into an electronic document format precisely reproducing the original image means, for example, conversion into the pdf format. Conversion into such a format prevents easy data tampering of conventional charts.

Upon a request for viewing the charts of a specific patient via the control server 1 or the consultation terminal 3, the electronic charts are read from the electronic chart file 11, and conventional chart are read from the conventional chart file 12 as well. Therefore, both types of charts of the specific patient can be read with the control server 1 or the consultation terminal 3.

Incidentally, the control server 1 may be configured such that among the information described in the conventional chart, those corresponding to the electronic chart information is directly input into the database 16 and the database overview generation unit 18 generates a time-series list so that the combination of respective items of the information input into the database 16 and the temporal variation of each information can be compared with the temporal variation of the graph.

Thus, information described in conventional charts can also be reflected in the list, whereby reducing the work load due to dual custody of conventional charts and new electronic charts when the electronic medical information system according to the present invention is introduced in a hospital or the like maintaining conventional charts.

**Fig. 12a** illustrates a database overview screen 180. On the left side of the database overview screen 180, there are provided a doctor in attendance display field 180a ("Naniwa University Hospital, Taro Kobe" in this example), a patient code/patient name display field 180b ("Hanako Osaka" in this example), a chief disease name display field 180c ("chronic sinusitis, diarrhea" in this example)

At the bottom of the chief disease name display field 180c is displayed a human body image 180d. In this human body image 180d, link buttons 180e (interview S) and 180f (interview O) are displayed respectively at the region of the patient's chief complaint (interview S) and the region where the symptom pointed out by the doctor's consultation (interview O) is developing, as will be described below. By clicking on link button 180e or 180f with the mouse, the chief complaint information or the consultation information relating to the region indicated by the link button 180e or 180f can be read from the database 16 for viewing.

Thus, owing to the human body image 180d, the development point can be grasped on a single screen when a plurality of symptoms is developing, thereby improving visibility of the symptoms. In addition, since the database overview, the chief complaint information and consultation information can be easily referred to point by point using the link button 180e or 180f in the human body image 180d, it becomes possible to smoothly view necessary information when preparing the therapeutic plan.

Furthermore, although not illustrated, a schematic drawing of focal region of disease may be displayed in the human body image 180d. For example, in the case of lung cancer, in addition to the link buttons 180e and 180f being displayed at the position of the lungs in the human body image 180d, a schematic drawing of the lungs may be displayed in half-tone, with the region of cancer reversed on the background.

With the help of displaying in the manner described above, a general overview, on the database overview screen 180, of the inspection result such as X-ray or CT scanning is facilitated, whereby the medical condition can be grasped faster, improving visibility as well.

Here, the displayed focal region itself may be symbolized as a link button, which may be clicked in with the mouse to display the actual image of X-ray or CT scanning.

At the bottom of the human body image 180d, there is provided a patient code input field 180g. The database overview of the patient corresponding to the input patient code may be displayed by inputting the patient code into the patient code input field 180g.

An icon menu 180h is displayed at the upper right of the database overview screen 180. Detailed description will be omitted because it is similar to that of the icon menu of Fig. 4a.

A progress graph 181 is displayed under the icon menu 180h. The progress graph 181 has consultation date as the horizontal axis 181a and scores (aggregated scores of the degree of the symptom which has been input as chief complaint information and aggregated scores of the degree of the symptom which has been input as consultation information) as the vertical axis 181 b. The aggregation of the scores is performed by transferring the scores of respective symptoms of the chief complaint information and consultation information from the database 16 to the score calculation unit 17, and adding them in the score calculation unit 17.

Polygonal line graphs 181c and 181d are formed by connecting the aggregated scores for each consultation date. The series of tasks from aggregation of the scores to formation of the progress graph 181 is automatically executed in the database overview generation unit of the control server 1.

Here, the field for the consultation date 09.24 is left as a blank. This indicates that although the next reservation information 120 has been input into the electronic chart, the patient did not actually appear on that date. The doctor determines the next reservation based on therapy, amount of prescribed medicine or the like. Therefore, the effect (such as drug effect) expected in the therapeutic plan may not be sufficiently obtained if the patient did not appear according to the reservation. Thus, the therapeutic plan has to be prepared again. In such a case, creating the progress graph 181 with the no-show date deleted may prevent grasping of correct course of the chief complaint and consultation, providing only insufficient information to contribute to replanning therapeutic plan. Therefore it has been determined to leave the no-show date in the progress graph 181.

Note that, if patient is receiving consultation for a plurality of different diseases around the same period, the polygonal lines of the progress graph 181 may be provided with different colors, for example, corresponding to respective diseases so that the progress graph 181 can be viewed with the diseases being distinguishable on the same screen (not shown).

Since the progress graph 181 displays the latest data, it is impossible to display all the progresses from the start of the therapy due to limitation of space on the screen. In the present embodiment, with the number of days displayable on the progress graph 181 being 7 days, the progress graph 181 is configured so that a past progress graph 181 can be further viewed by clicking on the scroll box 181e with the mouse.

However, in the case of a therapy lasting for a long period, viewing the past progress graph 181 using the scroll box 181e is troublesome and lacks visibility.

Thus, a past data list display button 181f is provided to allow the viewer to move to a progress graph listing screen 1811 shown in Fig. 12b, by clicking on the button with the mouse.

At the bottom of the progress graph listing screen 1811 is provided a period input field 1811a. By inputting the period desired for viewing into the period input field 1811 a and clicking on the listing button 1811 b with the mouse, the progress graph of the desired period input in the period input field 181 a can be displayed.

Returning to Fig. 12a, a dosage display field 182 displaying the variation of dosage in time series so that it can be compared with the temporal variation of the progress graph 181 is provided under the progress graph 181.

A dosage variation table 182a of a dosage display field 182 indicates, in the present embodiment, an administration period of each medicine prescribed according to the date of consultation in the progress graph 181 with respective lines.

Here, by clicking on a dosage information detail viewing button 182b with the mouse, dosage information in the electronic chart is read from the database 16 and details of the dosage information is displayed.

Under the dosage display field 182 is provided an injection/instillation display field 183 indicating the variation of injection/instillation in time series so that it can be compared with the temporal variation of the progress graph 181.

In the present embodiment, the injection/instillation variation table 183a of the injection/instillation display field 183 shows, with a mark (white circle), days on which injections/instillations are performed according to the date of the consultation in the progress graph 181.

Here, by clicking on the injection/instillation information detail viewing button 183b with the mouse, injection/instillation information in the electronic chart is read from the database 16 and details of the injection/instillation information is displayed.

Under the injection/instillation display field 183, there is provided a vital information display field 184 indicating the variation of vital information in time series so that it can be compared with the temporal variation of the progress graph 181.

A vital variation information table 184a of the vital information display field 184 displays measured values (not shown), respectively, according to the date of consultation in the progress graph 181.

Here, by clicking on a vital detail viewing button 184b with the mouse, vital information is read from the database 16 and details of the vital information is displayed.

Under the vital information display field 184 is provided an inspection display field 185 indicating the variation of inspection information in time series so that it can be compared with the temporal variation of the progress graph 181.

In the present embodiment, an inspection variation table 185a of the inspection display field 185 shows, with a mark (white circle), days on which inspections are performed according to the date of the consultation in the progress graph 181.

Here, by clicking on an inspection information detail viewing button 185b with the mouse, inspection information in the electronic chart is read from the database 16 and details of the inspection information is displayed.

Under the inspection display field 185, there is provided a chief complaint (interview S) display field 186 indicating the variation of chief complaint in time series so that it can be compared with the temporal variation of the progress graph 181.

In the present embodiment, a chief complaint variation table 186a of the chief complaint (interview S) display field 186 displays the degree of chief complaint evaluated by "-", "±", "+" and "++" for each consultation day in the progress graph 181.

Here, by clicking on a chief complaint information detail viewing button 186b with the mouse, chief complaint information is read from the database 16 and details of the chief complain information is displayed.

Under the chief complaint (interview S) display field 186, there is provided a chief complaint (interview S) display field 187 indicating the variation of chief complaint in time series so that it can be compared with the temporal variation of the progress graph 181.

In the present embodiment, a consultation variation table 187a of the chief complaint (interview O) display field 187 displays the degree of chief complaint evaluated by "-", "±", "+" and "++" for each consultation day in the progress graph 181.

Here, by clicking on a consultation information detail viewing button 186b with the mouse, consultation information is read from the database 16 and details of the consultation information is displayed.

The progress graph 181, the dosage variation table 182a, the injection/instillation variation table 183a, the vital variation information table 184a, the inspection variation table 185a, the chief complaint variation table 187a, the consultation variation table 187a of the database overview screen 180 are automatically generated in the database overview generation unit 18 by transferring their data from the database 16 to the database overview generation unit 18 which in turn processes the data.

Note that, in the present embodiment, the database overview screen 180 is composed of the dosage display field 182, the injection/instillation display field 183, the vital information display field 184, the inspection display field 185, the chief complaint (interview S) display field 187, the consultation (interview S) display field 187 so as to facilitate comparison with the temporal variation of the progress graph 181, it is by no means limited to this arrangement provided that it does not depart from the scope of the claims of the present invention. Thus, for example, the database overview screen 180 may include the progress graph 181 only, or alternatively, may be composed of the progress graph 181, the chief complaint (interview S) display field 187, and the consultation (interview S) display field 187.

By the way, although the above description has been provided assuming that the invention is arranged as a system, it may be in the form of a program which can be installed in a computer, or computer readable recording media having the program recorded thereon such CD-ROMs, MOs or FDs, for example, as long as the functionality can be realized on the computer.

Furthermore, the program may be recorded in a program server or the like connected to a communication network, and read into a computer via the communication network, at the time of use, to be recorded in the computer or an external storage device and executed as needed.

### Industrial Applicability

The present invention is available for generating and managing electronic medical information such as charts using a computer.

## Claims

1. An electronic medical information system using a computer to manage electronic medical information such as charts, said electronic medical information system being equipped with a control server comprising:
an input means for inputting, among the information written on said charts, patient's chief complaint information into a chief complaint information file and for inputting doctor's consultation information associated with said patient's chief complaint information into a consultation information file;
an accumulation means for accumulating said chief complaint information and said consultation information;
a calculation means for scoring, with respect to each date of consultation, the latest chief complaint information and consultation information input by said input means, and the past chief complaint information and consultation information accumulated by said accumulation means, respectively; and
a generation means for automatically generating, based on said scores, a list by which a temporal variation of said chief complaint information and consultation information can be viewed.

2. The electronic medical information system according to claim 1, wherein said control server comprises a display means for displaying said list to be viewable.

3. The electronic medical information system according to claim 1 or 2, wherein
said control server is configured to set and display a table in which a plurality of symptoms and degrees of respective symptoms are selectably listed, and the input means of said chief complaint information and consultation information automatically inputs one or more relevant symptoms and degrees of symptoms, in response to the user operation of selecting them from the table, into said chief complaint information file and the consultation information file, and wherein
said calculation means is configured to automatically calculate by aggregating relevant scores corresponding to the degree of the selected symptom, with each of said degrees of symptoms having a pre-defined score assigned thereto.

4. The electronic medical information system according to any of claims 1 to 3, wherein said control server is configured to display, in conjunction with said table, an image simulating a human body with check buttons provided on major regions thereof, and when any one of said symptoms is selected, the check button corresponding to the region which is developing the selected symptom is automatically checked.

5. The electronic medical information system according to any of claims 1 to 4, wherein said list generation means is configured to automatically generate a graph by which temporal variation of the scores calculated by said calculation means can be viewed.

6. The electronic medical information system according to any of claims 1 to 5, wherein said list generation means is configured to present said scores calculated by said calculation means in time series for respective symptoms of the chief complaint information and consultation information so that it can be compared with the temporal variation of said graph.

7. The electronic medical information system according to any of claims 1 to 6, wherein said control server comprises: a display means for displaying an image simulating a human body in conjunction with said list and displaying, when symptoms of the chief complaint information and consultation information are input by said input means, link buttons at the region where the respective symptoms are developing on the image simulating the human body; a reading means for reading, from the chief complaint information or consultation information accumulated in said accumulation means, each information of the region displaying the link button when the displayed link button is pressed; and a display means for displaying each information which has been read to be viewable.

8. The electronic medical information system according to any of claims 1 to 7, wherein said control server comprises a generation means for generating a list so that, when the patient is receiving consultation for a plurality of different diseases around the same period, the respective diseases can be viewed on the same screen in a distinguishable manner.

9. The electronic medical information system according to any of claims 1 to 8, wherein said control server comprises: an input means for inputting electronic chart information composed of one or more of information as to disease name, chief complaint reported at the consultation, history of present illness, anamnesis, anamneses of family members, observations of the doctor, inspection, treatment, dosage, injection/instillation, next-reservation; a generation means for generating an electronic chart based on the input information; a storage means for storing the generated electronic chart into an electronic chart file; a reading means for setting and displaying, on said list, a link button associated with the electronic chart and reading the electronic chart from said electronic chart file when the link button is pressed; and a display means for displaying the electronic chart which has been read out to be viewable.

10. The electronic medical information system according to any of claims 1 to 9, wherein the input means for inputting said electronic chart information is configured to input handwritten information either in an imaged or coded manner.

11. The electronic medical information system according to any of claims 1 to 10, wherein the input means for inputting said electronic chart information comprises: an audio input means, such as a microphone, for inputting audio information; and an image input means such as a camera for inputting image information, and wherein said electronic chart generation means generates an electronic chart so that the audio information and image information can be reproduced from the electronic chart as a part of the electronic chart.

12. The electronic medical information system according to any of claims 1 to 11, wherein if the patient did not appear on the day reserved in the next-reservation information which has been input by said input means of electronic chart information, said list generation means generates the list after clearing the field for the day.

13. The electronic medical information system according to any of claims 1 to 12, wherein said control server comprises: a reading means for reading, from said electronic chart file, one or more of information as to inspection, treatment, dosage, and injection/instillation; and an accumulation means for accumulating the information which has been read out, and wherein said control server sets/displays a link button for each information on the list when said list generation means arranges the temporal variation of each of the accumulated information in time series so that it can be compared with the temporal variation of the said graph, reads corresponding information accumulated by said accumulation means upon pressing of any of the link buttons, and display the information which has been read out to be viewable.

14. The electronic medical information system according to any of claims 1 to 13, wherein said control server comprises: a storing means for storing, into a conventional chart file, a conventional chart converted into an electronic document format which precisely reproduces an original image; a reading means for reading, upon request for viewing the conventional chart file, a conventional chart requested for viewing from said conventional chart file; and a display means for displaying the conventional chart which has been read out to be viewable.

15. The electronic medical information system according to any of claims 1 to 14, wherein, of the information recorded in said conventional chart file, the information corresponding to said electronic chart information is accumulated by the accumulation means according to claim 13, and said list generation means is configured to present the temporal variation of each information of the accumulated conventional chart in time series so that it can be compared with the temporal variation of said graph.

16. The electronic medical information system according to any of claims 1 to 15, wherein said input means of chief complaint information is implemented by transmitting data, to the control server, from a communication terminal used by the doctor for consultation via a LAN or a WAN, or from the patient's communication terminal via a communication network such as the Internet.

17. The electronic medical information system according to any of claims 1 to 16, wherein said input means of consultation information is implemented by transmitting data from said consultation terminal to said control server.

18. The electronic medical information system according to any of claims 1 to 17, wherein said patient's communication terminal transmits, via a communication network such as the Internet, vital information including one or more of the patient's weight, blood pressure, frequency of meals, content of meals, taking exercise or not, and sleeping hours to said control server, with said control server comprising an input means for receiving the transmitted vital information and inputting it into a vital information file, and a generation means for generating said list by arranging the vital information in time series so that it can be compared with the temporal variation of said graph.

19. The electronic medical information system according to any of claims 1 to 18, wherein said input means of electronic chart information is implemented by transmitting data from said consultation terminal to said control server via a LAN or a WAN.

20. The electronic medical information system according to any of claims 1 to 19, wherein said consultation terminal comprises an input means for inputting handwritten information in an imaged or coded form.

21. The electronic medical information system according to any of claims 1 to 20, wherein said consultation terminal comprises said audio input means and said image input means.

22. The electronic medical information system according to any of claims 1 to 21, wherein, upon receiving a request, via a communication network such as a LAN, a WAN, or the Internet, for viewing said list or the information accumulated by said accumulation means from said consultation terminal, said patient's communication terminal, and a communication terminal of a viewer such as an inspection engineer, a dispensing pharmacy clerk, an ambulance crew, a paramedic, a doctor on duty, or a doctor of another hospital, said control server is configured to display the list or said each information to be viewable, within the authorized range, from the consultation terminal, the patient's communication terminal, and the viewer's communication terminal, respectively.

23. The electronic medical information system according to any of claims 1 to 22, wherein said control server is configured such that, by letting an IC card reader/writer connected to a communication terminal read the IC card carried by a person permitted to use the communication terminal being one or more of said consultation terminal, said patient's communication terminal, and the viewer's communication terminal, said control server receives a signal requesting authorization via a communication network such as a LAN, a WAN, or the Internet and performs said authorization.

24. The electronic medical information system according to any of claims 1 to 23, wherein said control server is configured such that, by letting the reader/writer connected to the communication terminal of a viewer such as an ambulance crew, a paramedic, or a doctor on duty read said IC card carried by the patient and the IC card carried by the ambulance crew, the paramedic, or the doctor on duty, in case of emergency, the control server receives and authorizes a signal requesting authorization via a communication network such as a LAN, a WAN, or the Internet, and displays at least said information of history of present illness and anamnesis so that they can be viewed with the viewer's communication terminal.

25. An electronic medical information program for causing, for the purpose of introducing electronic medical information, a computer to function as: an input means for inputting said patient's chief complaint information into its chief complaint information file and for inputting said doctor's consultation information into its consultation information file; an accumulation means for accumulating the chief complaint information input into the chief complaint information file and the consultation information input into the consultation information file; a calculation means for scoring, with respect to each date of consultation, the input latest chief complaint information and consultation information, and the accumulated past chief complaint information and consultation information, respectively; a generation means for automatically generating, based on the calculated scores, a list by which the temporal variation of the chief complaint information and consultation information can be viewed.

26. The electronic medical information program according to claim 25 for causing, for the purpose of introducing electronic medical information, a computer to function as a display means for displaying said list.

27. The electronic medical information program according to claim 25 or 26 for causing, for the purpose of introducing electronic medical information, a computer to function as: an input means for inputting said electronic chart information; a generation means for generating an electronic chart from the input electronic chart information; a storing means for storing the generated electronic chart in an electronic chart file; a reading means for setting/displaying link buttons associated with the electronic chart file on said list and reading, upon pressing of any of the link buttons, the electronic chart from said electronic chart file; and a display means for displaying the electronic chart which has been read out to be viewable.

28. The electronic medical information program according to any of claims 25 to 27 for causing, for the purpose of introducing electronic medical information, a computer to function as: a reading means for reading, from said electronic chart file, one or more of information as to inspection, treatment, dosage, and injection/instillation; an accumulation means for accumulating the information which has been read out; said list generation means for arranging the temporal variation of each of the accumulated information in time series so that it can be compared with the temporal variation of said graph; a reading means for setting/displaying a link button for each information on the list and reading the corresponding information accumulated by said accumulation means upon pressing of any of the link buttons; and a display means for displaying the information which has been read out to be viewable.

29. The electronic medical information program according to any of claims 25 to 28 for causing, for the purpose of introducing electronic medical information, a computer to function as: a storage means for storing, into a conventional chart file, said conventional chart converted into an electronic document format which precisely reproduces an original image; a reading means for reading, upon request for viewing the conventional chart file, a conventional chart requested for viewing from said conventional chart file; and a display means for displaying the conventional chart which has been read out to be viewable.

30. The electronic medical information program according to any of claims 25 to 29 for causing, for the purpose of introducing electronic medical information, a computer to function as: an accumulation means for accumulating, using the accumulation means according to claim 28, the information corresponding to said electronic chart of the information recorded in said conventional chart file; and a generation means for generating said list by arranging the temporal variation of each information of the accumulated conventional chart in time series so that it can be compared with the temporal variation of said graph.

31. The electronic medical information program according to any of claims 25 to 30 for causing, for the purpose of introducing electronic medical information, a computer to function as: an input means for receiving, upon transmission of vital information including at least one or more of information of the patient's weight, blood pressure, frequency of meals, content of meals, taking exercise or not, sleeping hours, from said patient's communication terminal to the computer via a communication network such as the Internet, the transmitted vital information and inputting it into a vital information file; and a generation means for generating said list by arranging the vital information in time series so that it can be compared with the temporal variation of said graph.

32. A computer readable recording media for storing the electronic medical information program according to any of claims 25 to 31.
